# EUROPEAN PATENT APPLICATION

(11) **EP 4 130 046 A1**
(43) Date of publication of application: **08.02.2023**
(21) Application number: 21782157.8
(22) Date of filing: 30.03.2021
(51) Int. Cl.: C07K 16/46, C12N 15/13, C12N 15/63, C12N 5/10, A61K 39/395, A61P 35/00

(54) **PLATFORM FOR CONSTRUCTING MULTISPECIFIC ANTIBODY**

(30) Priority: 31.03.2020 CN 202010244571
(71) Applicant: Biotheus Inc., Tangjiawan Town, Xiangzhou District Zhuhai, Guangdong 519080 (CN)
(72) Inventor: LIU, Xiaolin, Zhuhai, Guangdong 519080 (CN); TSUN, Andy, Zhuhai, Guangdong 519080 (CN); SUN, Tsoyue Joanne, Zhuhai, Guangdong 519080 (CN); MIAO, Xiaoniu, Zhuhai, Guangdong 519080 (CN); WANG, Tao, Zhuhai, Guangdong 519080 (CN); DAI, Shuang, Zhuhai, Guangdong 519080 (CN)
(74) Representative: Latscha Schöllhorn Partner AG
(86) International application number: PCT/CN2021/084198
(87) International publication number: WO 2021/197359

(57) **Abstract**

Provided in the present invention is a method for constructing a multispecific antibody. The method comprises the steps of:(i)constructing a first polynucleotide and a second polynucleotide, respectively, wherein the first polynucleotide and the second polynucleotide respectively encode a first polypeptide containing a CL region and a second polypeptide containing a CH1 region, and a disulfide bond may be formed between the CL region of the first polypeptide and the CH1 region of the second polypeptide, such that the antibody has a heterodimeric form; and (ii) expressing the first polynucleotide and the second polynucleotide to obtain the first polypeptide and the second polypeptide, and dimerize the first polypeptide and the second polypeptide to form a multispecific antibody with a heterodimeric form. The antibody of the present invention can simultaneously bind to different targets and maintain the binding activity of the original antibody, which plays a role when the target is a membrane surface receptor or a target in a solution and has a biological activity against multiple targets.

## Description

### TECHNICAL FIELD

The present invention belongs to the biomedical or biopharmaceutical technical field, and particularly relates to a platform for construcing multispecific antibody.

### BACKGROUND

The original concept of bispecific antibodies (bispecific antibodies) was first proposed in 1960 by Nisonoff and his collaborators at the Roswell Park Memorial Institute in New York. Later, with the development of milestones in the fields of antibody engineering and antibody biology, the concept and technology for construction of bispecific antibodies is continuously innovative. There are currently over 100 bispecific antibody structural patterns, of which about one quarter have been developed as a technical platform and commercialized by biotechnology companies and pharmaceutical companies for novel antibody therapeutics. To date, more than 20 different commercial technology platforms have been available for the development of bispecific antibodies, and over 85 bispecific antibodies are in clinical development.

The impressive clinical outcome of a T-cell engaging bispecific antibody, blinatumomab, (targeting CD3 and CD19, approved by FDA in 2014 for the treatment of acute B-lymphocytic leukemia) has sparked interest and investment in this concept in the industry. Currently over 40 T-cell redirecting bispecific antibodies are in clinical development for the treatment of hematologic and solid tumors. In addition to cancer, inflammatory diseases have also been the focus of clinical development of bispecific antibodies. Roche's emicizumab (targeting coagulation factorX and factor IXa) was approved by FDA in November, 2017, making hemophilia the first non-cancer indication for bispecific antibodies. Currently, many teams are also exploring the therapeutic potential of bispecific antibodies in other disease areas, such as diabetes, HIV infection, other viral and bacterial infections, Alzheimer's disease, osteoporosis, etc. The dual-targeting feature of bispecific antibodies (i.e., the ability to specifically target two antigens or two different epitopes of one antigen simultaneously) makes them promising therapeutic opportunities, but the conversion of bispecific antibodies to clinical therapies remains challenging.

Therapeutic bispecific antibodies are a rapidly expanding diverse population of molecules. While the increased complexity of the dual-targeting concept compared to monoclonal antibodies presents additional challenges at different stages of discovery and development, bispecific antibodies provide exciting opportunities for the design and development of new drugs. From the perspective of disease field, current data shows that the industry is more looking forward to the treatment of cancer with bispecific antibodies. The continuous development of bispecific antibodies will have a lasting impact on the treatment of diseases such as cancer.

Therefore, there is an urgent need in the art to develop a method of constructing a multispecific antibody with low cost and high efficiency.

### SUMMARY

The present invention aims to provide a method of constructing a multispecific antibody with low cost and high efficiency.

In a first aspect of the present invention, there is provided a method for construction of a multispecific antibody, comprising the steps of:
(i) constructing a first polynucleotide encoding a first polypeptide having a structure represented by Formula I from N-terminus to C-terminus and a second polynucleotide encoding a second polypeptide having a structure represented by Formula II from N-terminus to C-terminus,

   A1-L1-B1-L2-CL-L3-A2 (Formula I)

   A3-L4-B2-L5-CH1-L6-A4 (Formula II)

   wherein,
   A1, A2, A3, and A4 are each independently an antibody or antigenic fragment thereof that targets a target of interest, and the target antigens targeted by each of A1, A2, A3, and A4 can be the same or different;
   L1, L2, L3, and L4 are each independently a null or linker element;
   B1 and B2 are both null, or B1 and B2 are the VL region and VH region, respectively, of an antibody targeting the same target;
   and a disulfide bond may be formed between the CL region of the first polypeptide and the CH1 region of the second polypeptide, such that the antibody has a heterodimeric form;
(ii) expressing the first polynucleotide and the second polynucleotide to obtain the first polypeptide and the second polypeptide, and dimerizing the first polypeptide and the second polypeptide to form a multispecific antibody with a heterodimeric form.

In another preferred example, the CL region of the first polypeptide has an amino acid sequence as set forth in SEQ ID NO. 9 or an amino acid sequence having a sequence identity greater than or equal to 85% (preferably 90%, more preferably 95%, 96%, 97%, 98%, or 99%) to the sequence as set forth in SEQ ID NO. 9.

In another preferred example, the CH1 region of the second polypeptide has an amino acid sequence as set forth in SEQ ID NO. 3 or an amino acid sequence having a sequence identity greater than or equal to 85% (preferably 90%, more preferably 95%, 96%, 97%, 98%, or 99%) to the sequence as set forth in SEQ ID NO. 3.

In another preferred example, the target of interest in A1, A2, A3, and A4 is an antigen, a cell surface receptor, a ligand, or a cytokine.

In another preferred example, the target of interest in A1, A2, A3, and A4 includes, but is not limited to: PD-1, TIGIT, human serum albumin, VEGF, PD-L1, PD-L2, or 41BB.

In another preferred example, the antibody or antigen fragment thereof that targets the target of interest in A1, A2, A3, and A4 is a VHH chain of a nanobody, an antibody heavy chain variable region, an antibody light chain variable region, an antibody Fc fragment, or a combination thereof.

In another preferred example, the antibody or antigen fragment thereof that targets the target of interest in A1, A2, A3, and A4 includes but is not limited to: a VHH chain of an anti-TIGIT nanobody, a VHH chain of an anti-HSA nanobody, a VHH chain of an anti-PD-L1 nanobody, a VHH chain of an anti-PD-L2 nanobody, a VH chain of an anti-VEGF antibody, a VL chain of an anti-VEGF antibody, a VH chain of an anti-PD-1 antibody, or a VL chain of an anti-PD-1 antibody.

In another preferred example, the VHH chain of the anti-TIGIT nanobody has an amino acid sequence as set forth in SEQ ID NO. 6 or an amino acid sequence having a sequence identity greater than or equal to 85% (preferably 90%, more preferably 95%, 96%, 97%, 98%, or 99%) to the sequence as set forth in SEQ ID NO. 6.

In another preferred example, the VHH chain of the anti-HSA nanobody has an amino acid sequence as set forth in SEQ ID NO. 5, or an amino acid sequence having a sequence identity greater than or equal to 85% (preferably 90%, more preferably 95%, 96%, 97%, 98%, or 99%) to the sequence as set forth in SEQ ID NO. 5.

In another preferred example, the VHH chain of the anti-PD-L1 nanobody has an amino acid sequence as set forth in SEQ ID NO: 14 or 28, or an amino acid sequence having a sequence identity greater than or equal to 85% (preferably 90%, more preferably 95%, 96%, 97%, 98%, or 99%) to the sequence as set forth in SEQ ID NO: 14 or 28.

In another preferred example, the VHH chain of the anti-PD-L2 nanobody has an amino acid sequence as set forth in SEQ ID NO. 19 or an amino acid sequence having a sequence identity greater than or equal to 85% (preferably 90%, more preferably 95%, 96%, 97%, 98%, or 99%) to the sequence as set forth in SEQ ID NO. 19.

In another preferred example, the antibodies targeting the same target of interest in B1 and B2 include, but are not limited to: an anti-PD-1 antibody, an anti-VEGF antibody.

In another preferred example, B 1 and B2 are the VL and VH regions, respectively, of an anti-PD-1 antibody; wherein the VL region of the anti-PD-1 antibody has an amino acid sequence as set forth in SEQ ID NO. 8 or an amino acid sequence having a sequence identity greater than or equal to 85% (preferably 90%, more preferably 95%, 96%, 97%, 98%, or 99%) to the sequence as set forth in SEQ ID NO. 8, and the VH region of the anti-PD-1 antibody has an amino acid sequence as set forth in SEQ ID NO. 2 or an amino acid sequence having a sequence identity greater than or equal to 85% (preferably 90%, more preferably 95%, 96%, 97%, 98%, or 99%) to the sequence as set forth in SEQ ID NO. 2.

In another preferred example, B 1 and B2 are the VL and VH regions, respectively, of an anti-VEGF antibody; wherein the VL region of the anti-VEGF antibody has an amino acid sequence as set forth in SEQ ID NO. 16 or an amino acid sequence having a sequence identity greater than or equal to 85% (preferably 90%, more preferably 95%, 96%, 97%, 98%, or 99%) to the sequence as set forth in SEQ ID NO. 16, and the VH region of the anti-VEGF antibody has an amino acid sequence as set forth in SEQ ID NO. 13 or an amino acid sequence having a sequence identity greater than or equal to 85% (preferably 90%, more preferably 95%, 96%, 97%, 98%, or 99%) to the sequence as set forth in SEQ ID NO. 13.

In another preferred example, the sequence of the linker element is (4GS)n, wherein n is a positive integer (e.g. 1, 2, 3, 4, 5, or 6), preferably n = 4.

In another preferred example, the sequence of the linker element is as set forth in SEQ ID NO: 4 or 21 or has greater than or equal to 85% (preferably 90%, more preferably 95%, 96%, 97%, 98%, or 99%) sequence identity to the sequence as set forth in SEQ ID NO: 4 or 21.

In another preferred example, the first polypeptide has an amino acid sequence as set forth in SEQ ID NO. 1 or an amino acid sequence having a sequence identity greater than or equal to 85% (preferably 90%, more preferably 95%, 96%, 97%, 98%, or 99%) to the sequence as set forth in SEQ ID NO. 1; and the second polypeptide has an amino acid sequence as set forth in SEQ ID NO. 7 or an amino acid sequence having a sequence identity greater than or equal to 85% (preferably 90%, more preferably 95%, 96%, 97%, 98%, or 99%) to the sequence as set forth in SEQ ID NO. 7.

In another preferred example, the first polypeptide has an amino acid sequence as set forth in SEQ ID NO. 10 or an amino acid sequence having a sequence identity greater than or equal to 85% (preferably 90%, more preferably 95%, 96%, 97%, 98%, or 99%) to the sequence as set forth in SEQ ID NO. 10; and the second polypeptide has an amino acid sequence as set forth in SEQ ID NO. 11, or an amino acid sequence having a sequence identity greater than or equal to 85% (preferably 90%, more preferably 95%, 96%, 97%, 98%, or 99%) to the sequence as set forth in SEQ ID NO. 11.

In another preferred example, the first polypeptide has an amino acid sequence as set forth in SEQ ID NO. 12 or an amino acid sequence having a sequence identity greater than or equal to 85% (preferably 90%, more preferably 95%, 96%, 97%, 98%, or 99%) to the sequence as set forth in SEQ ID NO. 12; and the second polypeptide has an amino acid sequence as set forth in SEQ ID NO. 15 or an amino acid sequence having a sequence identity greater than or equal to 85% (preferably 90%, more preferably 95%, 96%, 97%, 98%, or 99%) to the sequence as set forth in SEQ ID NO. 15.

In another preferred example, the first polypeptide has an amino acid sequence as set forth in SEQ ID NO. 17 or an amino acid sequence having a sequence identity greater than or equal to 85% (preferably 90%, more preferably 95%, 96%, 97%, 98%, or 99%) to the sequence as set forth in SEQ ID NO. 17; and the second polypeptide has an amino acid sequence as set forth in SEQ ID NO. 18 or an amino acid sequence having a sequence identity greater than or equal to 85% (preferably 90%, more preferably 95%, 96%, 97%, 98%, or 99%) to the sequence as set forth in SEQ ID NO. 18.

In another preferred example, the first polypeptide has an amino acid sequence as set forth in SEQ ID NO: 20 or an amino acid sequence having a sequence identity greater than or equal to 85% (preferably 90%, more preferably 95%, 96%, 97%, 98%, or 99%) to the sequence as set forth in SEQ ID NO: 20; and the second polypeptide has an amino acid sequence as set forth in SEQ ID NO. 18 or an amino acid sequence having a sequence identity greater than or equal to 85% (preferably 90%, more preferably 95%, 96%, 97%, 98%, or 99%) to the sequence as set forth in SEQ ID NO. 18.

In another preferred example, the first polypeptide has an amino acid sequence as set forth in SEQ ID NO. 22 or an amino acid sequence having a sequence identity greater than or equal to 85% (preferably 90%, more preferably 95%, 96%, 97%, 98%, or 99%) to the sequence as set forth in SEQ ID NO. 22; and the second polypeptide has an amino acid sequence as set forth in SEQ ID NO. 23 or an amino acid sequence having a sequence identity greater than or equal to 85% (preferably 90%, more preferably 95%, 96%, 97%, 98%, or 99%) to the sequence as set forth in SEQ ID NO. 23.

In another preferred example, the first polypeptide has an amino acid sequence as set forth in SEQ ID NO. 17 or an amino acid sequence having a sequence identity greater than or equal to 85% (preferably 90%, more preferably 95%, 96%, 97%, 98%, or 99%) to the sequence as set forth in SEQ ID NO. 17; and the second polypeptide has an amino acid sequence as set forth in SEQ ID NO. 24 or an amino acid sequence having a sequence identity greater than or equal to 85% (preferably 90%, more preferably 95%, 96%, 97%, 98%, or 99%) to the sequence as set forth in SEQ ID NO. 24.

In another preferred example, the first polypeptide has an amino acid sequence as set forth in SEQ ID NO. 17 or an amino acid sequence having a sequence identity greater than or equal to 85% (preferably 90%, more preferably 95%, 96%, 97%, 98%, or 99%) to the sequence as set forth in SEQ ID NO. 17; and the second polypeptide has an amino acid sequence as set forth in SEQ ID NO. 25 or an amino acid sequence having a sequence identity greater than or equal to 85% (preferably 90%, more preferably 95%, 96%, 97%, 98%, or 99%) to the sequence as set forth in SEQ ID NO. 25.

In a second aspect of the present invention, there is provided a multispecific antibody comprising a first polypeptide represented by Formula I from N-terminus to C-terminus and a second polypeptide represented by Formula II from N-terminus to C-terminus,

A1-L1-B1-L2-CL-L3-A2 (Formula I)

A3-L4-B2-L5-CH1-L6-A4 (Formula II)

wherein,
A1, A2, A3, and A4 are each independently an antibody or antigenic fragment thereof that targets a target of interest, and the target antigens targeted by each of A1, A2, A3, and A4 can be the same or different;
L1, L2, L3, and L4 are each independently a null or linker element;
B1 and B2 are both null, or B1 and B2 are the VL and VH regions, respectively, of an antibody targeting the same target;
and a disulfide bond may be formed between the CL region of the first polypeptide and the CH1 region of the second polypeptide, such that the antibody has a heterodimeric form.

In a third aspect of the present invention, there is provided a fusion protein comprising a multispecific antibody according to the second aspect of the present invention, and the first polypeptide in the multispecific antibody has a structure represented by Formula III from N-terminus to C-terminus,

A1-L1-CL-L3-Fc (Formula III)

wherein, Fc is a Fc fragment of an antibody, comprising a CH2 domain and a CH3 domain;
and the fusion protein may form a homodimer via disulfide bonding between Fc fragments.

In another preferred example, the first polypeptide has an amino acid sequence as set forth in SEQ ID NO. 27 or an amino acid sequence having a sequence identity greater than or equal to 85% (preferably 90%, more preferably 95%, 96%, 97%, 98%, or 99%) to the sequence as set forth in SEQ ID NO. 27; and the second polypeptide has an amino acid sequence as set forth in SEQ ID NO. 30 or an amino acid sequence having a sequence identity greater than or equal to 85% (preferably 90%, more preferably 95%, 96%, 97%, 98%, or 99%) to the sequence as set forth in SEQ ID NO. 30.

In a fourth aspect of the present invention, there is provided an isolated polynucleotide combination comprising a first nucleotide and a second nucleotide, wherein the first nucleotide encodes a first polypeptide of a multispecific antibody according to the second aspect of the present invention or of a fusion protein according to the third aspect of the present invention, and the second nucleotide encodes a second polypeptide.

In a fifth aspect of the present invention, there is provided a vector comprising a polynucleotide combination according to the fourth aspect of the present invention.

In another preferred example, the vector is selected from the group consisting of: DNA, RNA, viral vectors, plasmids, transposons, other gene transfer systems, or combinations thereof; preferably, the expression vector comprises a viral vector, such as lentivirus, adenovirus, AAV virus, retrovirus, or combinations thereof.

In a sixth aspect of the present invention, there is provided a host cell comprising a vector according to the fifth aspect of the present invention, or having incorporated into its genome a combination of polynucleotides according to the fourth aspect of the present invention;
alternatively, the host cell expresses a multispecific antibody according to the second aspect of the present invention or a fusion protein according to the third aspect of the present invention.

In another preferred example, the host cell comprises a prokaryotic cell or a eukaryotic cell.

In another preferred example, the host cell is selected from the group consisting of Escherichia coli, yeast cells, and mammalian cells.

In a seventh aspect of the present invention, there is provided a method of producing an antibody comprising the steps of:
(a) culturing a host cell according to the sixth aspect of the present invention under suitable conditions to obtain a culture comprising a multispecific antibody according to the second aspect of the present invention or a fusion protein according to the third aspect of the present invention; and
(b) purifying and/or isolating the culture obtained in step (a) to obtain the antibody.

In another preferred example, the purification may be performed by affinity chromatography purification and isolation to obtain the antibody of interest.

In another preferred example, the purity of the purified and isolated antibody of interest is greater than 95%, greater than 96%, greater than 97%, greater than 98%, greater than 99%, and preferably 100%.

In an eighth aspect of the present invention, there is provided an immunoconjugate comprising:
(a) a multispecific antibody according to the second aspect of the present invention or a fusion protein according to the third aspect of the present invention; and
(b) a coupling moiety selected from the group consisting of: a detectable label, a drug, a toxin, a cytokine, a radionuclide, or an enzyme, a gold nanoparticle/nanorod, a nano magnetic particle, a viral coat protein or VLP, or a combination thereof.

In another preferred example, the radionuclide includes:
(i) a diagnostic isotope selected from the group consisting of: Tc-99m, Ga-68, F-18, I-123, I-125, I-131, In-111, Ga-67, Cu-64, Zr-89, C-11, Lu-177, Re-188, or a combination thereof; and/or
(ii) a therapeutic isotope selected from the group consisting of: Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133 Yb-169, Yb-177, or a combination thereof.

In another preferred example, the coupling moiety is a drug or toxin.

In another preferred example, the drug is a cytotoxic drug.

In another preferred example, the cytotoxic agent is selected from the group consisting of: an anti-tubulin drug, a DNA minor groove binding agent, a DNA replication inhibitor, an alkylating agent, an antibiotic, a folate antagonist, an anti-metabolite drug, a chemotherapeutic sensitizer, a topoisomerase inhibitor, a vinca alkaloid, or a combination thereof.

Examples of particularly useful cytotoxic drugs include, for example, DNA minor groove binding agents, DNA alkylating agents, and tubulin inhibitors; typical cytotoxic drugs include, for example, auristatins, camptothecins, duocarmycins, etoposides, maytansinoids and maytansinoids (e.g., DM1 and DM4), taxanes, benzodiazepines, or benzodiazepine-containing drugs (e.g., pyrrolo [1,4] benzodiazepines (PBDs), indolinobenzodiazepines and oxazolidinobenzodiazepines), vinca alkaloids, or combinations thereof.

In another preferred example, the toxin is selected from the group consisting of: auristatins (e.g., auristatin E, auristatin F, MMAE, and MMAF), chlortetracycline, maytansinoids, ricin, ricin A-chain, combretastatin, duocarmycin, dolastatin, adriamycin, daunorubicin, paclitaxel, cisplatin, cc1065, ethidium bromide, mitomycin, etoposide, tenoposide, vincristine, vinblastine, colchicine, dihydroxy anthracin dione, actinomycin, diphtheria toxin, Pseudomonas Exotoxin (PE) A, PE40, abrin, abrin A-chain, modeccin A-chain, alpha-sarcina, gelonin, mitogeltin, restrictocin, phenomycin, enomycin, curcin, crotin, calicheamicin, *Sapaonaria officinalis* inhibitor, glucocorticoid, or a combination thereof.In another preferred example, the conjugating moiety is a detectable label.

In another preferred example, the conjugate is selected from the group consisting of: fluorescent or luminescent labels, radioactive labels, MRI (magnetic resonance imaging) or CT (computed tomography) contrast agents, or enzymes capable of producing detectable products, radionuclides, biotoxins, cytokines (e.g., IL-2), antibodies, antibody Fc fragments, antibody scFv fragments, gold nanoparticles/nanorods, viral particles, liposomes, nano magnetic particle, prodrug-activating enzymes (e.g., DT-diaphorase (DTD) or biphenyl hydrolase-like protein (BPHL)), chemotherapeutic agents (e.g., cisplatin).

In another preferred example, the immunoconjugate comprises a multivalent (e.g. bivalent) multispecific antibody according to the second aspect of the present invention. In another preferred example, the multivalent refers to that the amino acid of the immunoconjugate comprises multiple repeats of the antibody according to the second aspect of the present invention or a fusion protein according to the third aspect of the present invention.

In a ninth aspect of the present invention there is provided a use of a multispecific antibody according to the second aspect of the present invention, a fusion protein according to the third aspect of the present invention, or an immunoconjugate according to the eighth aspect of the present invention, in the manufacture of a medicament, a reagent, a detection plate, or a kit;
wherein the reagent, the detection plate or the kit is used for detecting the presence or absence of the target molecule of interest in the sample;
and the medicament is used for treating or preventing tumors expressing target molecules of interest.

In another preferred example, the conjugating moiety of the immunoconjugate is a diagnostic isotope.

In another preferred example, the reagent is one or more reagents selected from the group consisting of an isotope tracer, a contrast agent, a flow detection reagent, a cell immunofluorescence detection reagent, a nano-magnetic particle and a developing reagent.

In another preferred example, the agent for detecting a target molecule of interest in a sample is a contrast agent for (in vivo) detection of a target molecule of interest.

In another preferred example, the detection is an in vivo detection or an in vitro detection. In another preferred example, the detection includes flow detection and cell immunofluorescence detection.

In another preferred example, the tumor includes but is not limited to: acute myelocytic leukemia, chronic granulocytic leukemia, multiple myelopathy, non-Hodgkin lymphoma, colorectal cancer, breast cancer, carcinoma of large intestine, gastric cancer, liver cancer, leukemia, kidney tumor, lung cancer, small intestine cancer, bone cancer, prostatic cancer, prostatic cancer, cervical cancer, lymph cancer, adrenal gland tumor, and bladder tumor.

In a tenth aspect of the present invention, there is provided a pharmaceutical composition comprising: (i) a multispecific antibody according to the second aspect of the present invention, a fusion protein according to the third aspect of the present invention, or an immunoconjugate according to the eighth aspect of the present invention; and (ii) a pharmaceutically acceptable carrier.

In another preferred example, the conjugating moiety of the immunoconjugate is a drug, a toxin, and/or a therapeutic isotope.

In another preferred example, the pharmaceutical composition further comprises other drugs for treating tumors, such as cytotoxic drugs.

In another preferred example, the other drug for treating tumor comprises paclitaxel, doxorubicin, cyclophosphamide, axitinib, lenvatinib, or pembrolizumab.

In another preferred example, the pharmaceutical composition is used for treating tumors expressing target molecules of interest (i.e., positive for target molecules of interest).

In another preferred example, the pharmaceutical composition is injection dosage form. In another preferred example, the pharmaceutical composition is used for preparing a medicament for preventing and treating tumors.

In an eleventh aspect of the present invention, there is provided a method of treating a disease, the method comprising: administering a multispecific antibody according to the second aspect of the present invention, a fusion protein according to the third aspect of the present invention, an immunoconjugate according to the eighth aspect of the present invention, or a pharmaceutical composition according to the tenth aspect of the present invention to a subject in need thereof.

In another preferred example, the subject comprises a mammal, preferably a human.

In a twelfth aspect of the present invention, there is provided a kit comprising a multispecific antibody according to the second aspect of the present invention, a fusion protein according to the third aspect of the present invention, an immunoconjugate according to the eighth aspect of the present invention, or a pharmaceutical composition according to the tenth aspect of the present invention, and an instruction.

In another preferred example, the instruction describes that the kit is used for non-invasively detecting the expression of target molecules of interest in a subject for detection.

In another preferred example, the instruction describes that the kit is used for detecting tumors expressing target molecules of interest (i.e., positive for target molecules of interest).

It is to be understood that within the scope of the present invention, the above-described features of the present invention and those specifically described below (e.g., in the examples) may be combined with each other to form new or preferred embodiments. These will not be repeated due to the length of the specification.

### BRIEF DESCRIPTION OF THE DRAWINGS

FIG. 1 shows a schematic of the structures of three trispecific antibodies;
FIG. 2 shows the results of the antigen co-binding ability of an anti-PD-1/TIGIT/human serum albumin trispecific antibody determined using the Octet system;
FIG. 3 shows the results of the assay of the binding activity of an anti-PD-1/TIGTT/human serum albumin trispecific antibody to CHO-hPD-1 cells and CHO-hTIGIT cells;
FIG. 4 shows the binding activity of an anti-PD-1/TIGIT/human serum albumin trispecific antibody to human serum albumin as determined by ELISA;
FIG. 5 shows the blocking effect of an anti-PD-1/TIGIT/human serum albumin trispecific antibody on the binding of human PD-L1 to human PD-1;
FIG. 6 shows the blocking effect of an anti-PD-1/TIGIT/human serum albumin trispecific antibody on the binding of CD155 to TIGIT;
FIG. 7 shows the results of the antigen co-binding capacity of an anti-VEGF/PD-L1/human serum albumin trispecific antibody determined using the Octet system;
FIG. 8 shows the results of the assay of the binding activity of an anti-VEGF/PD-L1/human serum albumin trispecific antibody to CHO-hPD-1 cells;
FIG. 9 shows the binding activity of an anti-VEGF/PD-L1/human serum albumin trispecific antibody to human serum albumin as determined by ELISA;
FIG. 10 shows the binding activity of an anti-VEGF/PD-L1/human serum albumin trispecific antibody to human VEGF protein as determined by ELISA;
Figure 11 shows a schematic of the structures of three trispecific antibodies and one tetraspecific antibody;
FIG. 12 shows the results of the assay of the binding activity of three PD-L1/PD-L2/human serum albumin trispecific antibodies to CHO-hPD-L1 cells and CHO-hPD-L2 cells, and the results of the assay of the binding activity of an anti-PD-L1/PD-L2/TIGIT/human serum albumin tetraspecific antibody to CHO-hPD-L1 cells, CHO-hPD-L2 cells, and CHO-hTIGIT cells;

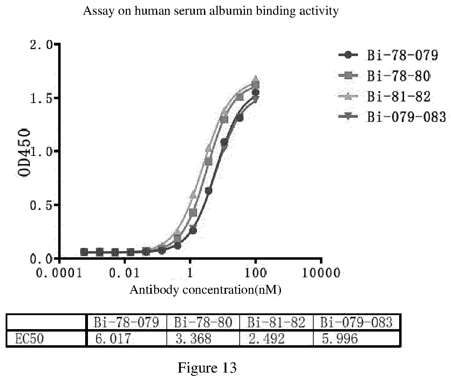

FIG. 13 shows the binding activity of three anti-PD-L1/PD-L2/human serum albumin trispecific antibodies and an anti-PD-L1/PD-L2/TIGIT/human serum albumin tetraspecific antibody to human serum albumin as determined by ELISA;
FIG. 14 shows the blocking effect of three anti-PD-L1/PD-L2/human serum albumin trispecific antibodies and an anti-PD-L1/PD-L2/TIGIT/human serum albumin tetraspecific antibody on the binding of human PD-L1 or PD-L2 to human PD-1;
FIG. 15 shows the results of three anti-PD-L1/PD-L2/human serum albumin trispecific antibodies and an anti-PD-L1/PD-L2/TIGIT/human serum albumin tetraspecific antibody simultaneously blocking PD-L1/PD-1 and PD-L2/PD-1 signaling pathways in vitro;
FIG. 16 shows a schematic of the structure of a trispecific antibody;
FIG. 17 shows the results of the assay of the binding activity of an PD-L1/41BB/human serum albumin trispecific antibody to CHO-hPD-L1 cells and CHO-41BB cells;
FIG. 18 shows the binding activity of a PD-L1/41BB/human serum albumin trispecific antibody to human serum albumin as determined by ELISA;
FIG. 19 shows the ability of an anti-PD-L1/41BB/human serum albumin trispecific antibody to bridge cells expressing PD-L1/41BB;
FIG. 20 shows the blocking effect of an anti-PD-L1/41BB/human serum albumin trispecific antibody on the binding of human PD-L1 to human PD-1;
FIG. 21 shows a schematic of the structure of the PD-L1/PD-L2 bispecific antibody Fc fusion protein as described in Example 5; and
FIG. 22 shows the results of assay of the binding activity of an anti-PD-L1/PD-L2 bispecific antibody Fc fusion protein to CHO-hPD-L1 cells and CHO-hPD-L2 cells.

### DETAILED DESCRIPTION

The present inventors have made extensive and intensive studies and, as a result, have developed a method for construction of a multispecific antibody for the first time through a large number of screenings. Experiments have demonstrated that stable heterodimers may be formed by linking antigen-binding fragments (such as antibody variable regions, single domain antibodies or Fc) to CL and CH1 fragments of the native antibodies. The multispecific antibody constructed by the method of the present Application is able to simultaneously bind to different targets and maintain the binding activity of the original antibody; is effective when the target is a membrane surface receptor or a target in a solution; has biological activity against multiple targets; and may link single domain antibodies or normal antibodies or Fc fragments. Therefore, the method and the provided platform of the present invention have huge application prospects. The present invention has been completed on the basis of this finding.

### Term(s)

In order that the disclosure may be more readily understood, certain terms are first defined. As used in this application, each of the following terms shall have the meaning given below, unless explicitly specified otherwise herein. Additional definitions are set forth throughout the application.

As used herein, the terms " platform for construcing multispecific antibody " and "construction methods of the present invention" are used interchangeably and refer to a method for construction of multispecific antibodies according to the first aspect of the present invention, wherein a heterodimer formed between CL and CH1 via disulfide bonding is the core structure and is fused with an antibody or antigenic fragment thereof targeting different target sites of interest.

### Multispecific antibodies

Bispecific/multispecific antibodies (BsAb, MsAb) are artificial proteins composed of fragments of two or more different monoclonal antibodies and thus can bind to two or more different types of antigens. For example, in cancer immunotherapy, engineered BsAbs bind both to cytotoxic cells and to the target to be killed (e.g., tumor cells). At least three types of bispecific antibodies have been proposed or tested, including trifunctional antibodies, chemically linked Fabs, and bispecific T cell adaptors. To overcome the difficulties in manufacturing, the first generation of BsMAbs, referred to as trifunctional antibodies, have been developed, consisting of two heavy chains and two light chains, each from different antibodies; the two Fab regions are directed to two antigens; The Fc region consists of two heavy chains and forms a third binding site, and thus the name is called.

Other types of bispecific antibodies have been designed to address certain problems, such as short half-life, immunogenicity, and side effects caused by cytokine release. They include: chemically linked Fabs, which consist of only Fab regions, and various types of divalent and trivalent single chain variable regions (scFvs) (which mimic the fusion protein of the two antibody variable domains). The most recently developed formats are bispecific T cell adaptors (BiTE) and tetrafunctional antibodies.

Antibodies are well known as a class of immunoglobulins that specifically bind to an antigen and are composed of four polypeptide chains, two chains of larger molecular weight being referred to as heavy chains (H chains) and two chains of smaller molecular weight being referred to as Light chains (L chains). In monoclonal antibodies, the amino acid composition of the two H-chains and the two L-chains is identical, whereas bispecific antibodies were developed by co-expressing two different H-chains and two different L-chains. Obtaining functional bispecific antibodies from 10 possible recombinant mixtures of H2L2 is one of the initial challenges in bispecific antibody development, commonly referred to as chain-related problems. Over the past decades, researchers have developed a number of strategies to address this problem.

### Fragment-based formats

The fragment-based bispecific antibody without an Fc region simply binds to a plurality of antibody fragments within one molecule, which avoids chain-related problems, and has the advantages of high yield and low cost and the disadvantage of a relatively short half-life. In addition, fragment-based bispecific antibodies may present stability and polymerization problems.

### Symmetric formats

The symmetric formats of bispecific antibodies retains the Fc region, which is closer to the native antibody, but differed in size and structure. These differences may negatively impact the beneficial properties associated with native antibodies (e.g., stability and solubility), which may compromise the physicochemical and/or pharmacokinetic properties of these bispecific antibodies.

### Asymmetric formats

Most bispecific antibodies with asymmetric formats are very similar to natural antibodies and are considered to have the potential for minimal immunogenicity. However, the complex engineering that may be involved to solve chain-related problems may offset this advantage of some asymmetricformats bispecific antibodies.

In the present invention, however, a class of multispecific antibodies based on heterodimeric forms is provided.

As used herein, the terms "multispecific antibody of the present invention", " multi-antibody of the present invention", and "antibody of the present invention" are used interchangeably and all refer to multispecific antibodies constructed using the methods for construction of multispecific antibodies provided by the present invention. Preferably, the antibody provided by the present invention comprises a first polypeptide as set forth in Formula I from N-terminus to C-terminus and a second polypeptide as set forth in Formula II from N-terminus to C-terminus,

A1-L1-B1-L2-CL-L3-A2 (Formula I)

A3-L4-B2-L5-CH1-L6-A4 (Formula II)

wherein,
A1, A2, A3, and A4 are each independently an antibody or antigenic fragment thereof that targets a target of interest, and the target antigens targeted by each of A1, A2, A3, and A4 can be the same or different;
L1, L2, L3 and L4 are each independently a null or linker element;
B 1 and B2 are both null, or B1 and B2 are the VL and VH regions, respectively, of an antibody targeting the same target of interest;
and a disulfide bond may be formed between the CL region of the first polypeptide and the CH1 region of the second polypeptide, such that the antibody has a heterodimeric form.

In a preferred embodiment, the CL region in Formula I has an amino acid sequence as set forth in SEQ ID NO. 9 and the CH1 region in Formula II has an amino acid sequence as set forth in SEQ ID NO. 3.

In one embodiment, the multispecific antibody is an anti-PD-1/TIGIT/human serum albumin trispecific antibody, wherein the first polypeptide has an amino acid sequence as set forth in SEQ ID NO. 1 and the second polypeptide has an amino acid sequence as set forth in SEQ ID NO. 7.

In one embodiment, the multispecific antibody is an anti-PD-1/TIGIT/human serum albumin trispecific antibody, wherein the first polypeptide has an amino acid sequence as set forth in SEQ ID NO. 10 and the second polypeptide has an amino acid sequence as set forth in SEQ ID NO. 11.

In one embodiment, the multispecific antibody is an anti-VEGF/PD-L1/human serum albumin trispecific antibody, wherein the first polypeptide has an amino acid sequence as set forth in SEQ ID NO. 12 and the second polypeptide has an amino acid sequence as set forth in SEQ ID NO. 15.

In one embodiment, the multispecific antibody is an anti-PD-L1/PD-L2/human serum albumin trispecific antibody, wherein the first polypeptide has an amino acid sequence as set forth in SEQ ID NO. 17 and the second polypeptide has an amino acid sequence as set forth in SEQ ID NO. 18.

In one embodiment, the multispecific antibody is an anti-PD-L1/PD-L2/human serum albumin trispecific antibody, wherein the first polypeptide has an amino acid sequence as set forth in SEQ ID NO. 20 and the second polypeptide has an amino acid sequence as set forth in SEQ ID NO. 18.

In one embodiment, the multispecific antibody is an anti-PD-L1/PD-L2/human serum albumin trispecific antibody, wherein the first polypeptide has an amino acid sequence as set forth in SEQ ID NO. 22 and the second polypeptide has an amino acid sequence as set forth in SEQ ID NO. 23.

In one embodiment, the multispecific antibody is an anti-PD-L1/PD-L2/TIGIT/human serum albumin tetraspecific antibody, wherein the first polypeptide has an amino acid sequence as set forth in SEQ ID NO: 17 and the second polypeptide has an amino acid sequence as set forth in SEQ ID NO: 24.

In one embodiment, the multispecific antibody is an anti-PD-L1/41BB/human serum albumin trispecific antibody, wherein the first polypeptide has an amino acid sequence as set forth in SEQ ID NO. 17 and the second polypeptide has an amino acid sequence as set forth in SEQ ID NO. 25.

In another embodiment, the present invention provides a fusion protein in which an Fc fragment is fused to the C-terminus of a first polypeptide in a multispecific antibody of the present invention, such that the multispecific antibody is capable of homodimerization due to disulfide bonding between the Fc fragments to form a more stable homodimer.

Preferably, the fusion protein is an anti-PD-L1/PD-L2 bispecific antibody, wherein the first polypeptide has an amino acid sequence as set forth in SEQ ID NO. 27 and the second polypeptide has an amino acid sequence as set forth in SEQ ID NO. 30.

As used herein, the terms "single domain antibody", "nanobody VHH", and "nanobody" have the same meaning, referring to a nanobody (VHH) consisting of only one heavy chain variable region constructed by cloning of the variable region of the heavy chain of an antibody, which is the smallest antigen-binding fragment with complete function. The nanobody (VHH) consisting of only one heavy chain variable region are typically constructed by obtaining an antibody naturally lacking the light chain and heavy chain constant region 1(CH1) and then cloning the variable region of the antibody heavy chain. As used herein, the term "variable" means that certain portions of the variable regions of an antibody differ in sequence, which results in the binding and specificity of each particular antibody against its particular antigen. However, the variability is not evenly distributed throughout the antibody variable region. It is concentrated in three fragments called Complementarity Determining Regions (CDRs) or hypervariable regions in the light and heavy chain variable regions. The more conserved portions of the variable regions are called Framework Regions (FR). The variable regions of native heavy and light chains each comprise four FR regions, which are in a substantially a beta-sheet configuration, connected by three CDRs that form a connecting loop, and in some cases may form part of a beta-sheet configuration . The CDRs in each chain lie closely together via the FR region and form the antigen binding site of the antibody with the CDRs of the other chain (see Kabat et al, NIH Publ. No. 91-3242, Vol. I, pages 647-669 (1991)). The constant regions are not directly involved in binding of the antibody to the antigen, but they exhibit different effector functions, such as participation in antibody-dependent cytotoxicity of the antibody.

As used herein, the term "framework region" (FR) refers to amino acid sequences inserted between CDRs, i.e., refers to those portions of the light and heavy chain variable regions of an immunoglobulin that are relatively conserved between different immunoglobulins in a single species. The light and heavy chains of immunoglobulins each have four FRs, designated as FR1-L, FR2-L, FR3-L, FR4-L and FR1-H, FR2-H, FR3-H, FR 4-H respectively. Accordingly, the light chain variable domain may thus be referred to as (FR1-L) - (CDR1-L) - (FR2-L) - (CDR2-L) - (FR3-L) - (CDR3-L) - (FR4-L) and the heavy chain variable domain may thus be referred to as (FR1-H) - (CDR1-H)-(FR2-H) - (CDR2-H) - (FR3-H) - (CDR3-H) - (FR 4-H). Preferably, the FR of the present invention is a human antibody FR or a derivative thereof that is substantially identical to a naturally-occurring human antibody FR, i.e., that has a sequence identity of 85%, 90%, 95%, 96%, 97%, 98%, or 99%.

Knowing the amino acid sequences of the CDRs, one skilled in the art can readily determine the framework regions FR1-L, FR2-L, FR3-L, FR4-L and/or FR1-H, FR2-H, FR3-H, FR 4-H.

As used herein, the term "human framework region" is a framework region that is substantially identical (about 85% or more, specifically 90%, 95%, 97%, 99%, or 100%) to the framework region of a naturally-occurring human antibody.

As used herein, the term "affinity" is theoretically defined by a balanced association between an intact antibody and an antigen. The affinity of the bispecific antibodies of the present invention may be assessed or determined by KD values (dissociation constants) (or other means of determination), such as Bio-layer interferometry (BLI), using FortebioRed96 instrument.

As used herein, the term "linker" refers to one or more amino acid residues inserted into an antibody of the present invention that provide sufficient mobility to each domain or region.

As known to those skilled in the art, immunoconjugates and fusion expression products include: drugs, toxins, cytokines, radionuclides, enzymes, and conjugates formed by binding other diagnostic or therapeutic molecules to the antibodies or fragments thereof of the present invention. The present invention also includes a cell surface marker or antigen that binds to the multispecific antibody or fragment thereof.

In the present invention, the terms "antibody of the present invention", "protein of the present invention", or "polypeptide of the present invention" are used interchangeably and refer to multispecific antibodies provided by the present invention, which may or may not contain the initial methionine.

The present invention also provides other proteins or fusion expression products having an antibody of the present invention. In particular, the present invention includes any protein or protein conjugate and fusion expression product (i.e., immunoconjugate and fusion expression product) having a heavy chain comprising a variable region, provided that the variable region is identical or at least 90% homologous, preferably at least 95% homologous, to the heavy chain variable region of an antibody of the present invention. The present invention includes not only intact antibodies, but also fragments of immunologically active antibodies or fusion proteins formed by the antibodies with other sequences. Accordingly, the present invention also includes fragments, derivatives, and analogs of the antibodies.

As used herein, the terms "fragment", "derivative", and "analog" refer to a polypeptide that retains substantially the same biological function or activity as an antibody of the present invention. A polypeptide fragment, derivative, or analogue of the present invention may be (i) a polypeptide in which one or more conserved or non-conserved amino acid residues (preferably conserved amino acid residues) are substituted, and such substituted amino acid residues may or may not be encoded by the genetic code, or (ii) a polypeptide having a substituent group in one or more amino acid residues, or (iii) a polypeptide formed by fusing the mature polypeptide to another compound (such as a compound that increases the half-life of the polypeptide, e.g., polyethylene glycol), or (iv) a polypeptide formed by fusing an additional amino acid sequence to the sequence of this polypeptide (such as a leader or secretory sequence or a sequence used to purify this polypeptide or a proprotein sequence, or a fusion protein with a 6His tag). Such fragments, derivatives, and analogs are well known to those skilled in the art in light of the teachings herein.

The antibodies of the present invention also include a form that have the same function as the antibodies of the present invention, and the first or second polypeptide of which has variation(s). These form of variation(s) include (but are not limited to): deletion, insertion, and/or substitution of one or more (usually 1 to 50, preferably 1 to 30, more preferably 1 to 20, most preferably 1 to 10) amino acids, and addition of one or several (usually up to 20, preferably up to 10, more preferably up to 5) amino acids at the C-terminus and/or N-terminus. For example, in the art, substitutions with amino acids that are similar or analogous in performance do not typically alter the function of the protein. Also, for example, addition of one or several amino acids at the C-terminus and/or N-terminus does not generally alter the function of the protein. The term also includes active fragments and active derivatives of the antibodies of the present invention.

Variants of the polypeptide include: homologous sequences, conservative variants, allelic variants, natural mutants, induced mutants, proteins encoded by DNA capable of hybridizing to DNA encoding the antibody of the present invention under high or low stringency conditions, and polypeptides or proteins obtained using antisera raised against the antibody of the present invention.

The present invention also provides other polypeptides, such as fusion proteins comprising single domain antibodies or fragments thereof. In addition to substantially full-length polypeptides, the present invention also encompasses fragments of the single domain antibodies of the present invention. Typically, the fragment has at least about 50 contiguous amino acids, preferably at least about 50 contiguous amino acids, more preferably at least about 80 contiguous amino acids, and most preferably at least about 100 contiguous amino acids of an antibody of the present invention.

In the present invention, "conservative variants of the antibody of the present invention" refers to a polypeptide in which up to 10, preferably up to 8, more preferably up to 5, and most preferably up to 3 amino acids are substituted with qualitatively similar or analogous amino acids compared to the amino acid sequence of the antibody of the present invention. These conservative variant polypeptides are preferably produced by amino acid substitutions according to Table A.

**TABLE A**

| **Initial residue** | **Representative substitutions** | **Preferred substitutions** |
|---|---|---|
| **Ala (A)** | Val; Leu; Ile | Val |
| **Arg (R)** | Lys; Gln; Asn | Lys |
| **Asn (N)** | Gln; His; Lys; Arg | Gin |
| **Asp (D)** | Glu | Glu |
| **Cys (C)** | Ser | Ser |
| **Gln (Q)** | Asn | Asn |
| **Glu (E)** | Asp | Asp |
| **Gly (G)** | Pro; Ala | Ala |
| **His (H)** | Asn; Gin; Lys; Arg | Arg |
| **Ile (I)** | Leu; Val; Met; Ala; Phe | Leu |
| **Leu (L)** | Ile; Val; Met; Ala; Phe | Ile |
| **Lys (K)** | Arg; Gln; Asn | Arg |
| **Met (M)** | Leu; Phe; Ile | Leu |
| **Phe (F)** | Leu; Ual; Ile; Ala; Tyr | Leu |
| **Pro (P)** | Ala | Ala |
| **Ser (S)** | Thr | Thr |
| **Thr (T)** | Ser | Ser |
| **Trp (W)** | Tyr; Phe | Tyr |
| **Tyr (Y)** | Trp; Phe; Thr; Ser | Phe |
| **Val (V)** | Ile; Leu; Met; Phe; Ala | Leu |

The present invention also provides polynucleotide molecules encoding the above antibodies or fragments or fusion proteins thereof. The polynucleotide of the present invention may be in the form of DNA or RNA. The form of DNA includes cDNA, genomic DNA or artificially synthesized DNA. The DNA may be single-stranded or double-stranded. The DNA may be the coding strand or the non-coding strand.

The polynucleotides encoding the mature polypeptides of the present invention include: a coding sequence encoding only the mature polypeptide; the coding sequence for the mature polypeptide and various additional coding sequences; the coding sequence for the mature polypeptide (and optionally additional coding sequences) as well as non-coding sequences.

The term "polynucleotide encoding a polypeptide" may include a polynucleotide encoding the polypeptide, and may also include a polynucleotide for additional coding and/or non-coding sequences.

The present invention also relates to polynucleotides which hybridize to the above-described sequences and which have at least 50%, preferably at least 70%, and more preferably at least 80% identity between the two sequences. The present invention particularly relates to polynucleotides which can hybridize to the polynucleotides of the present invention under stringent conditions. In the present invention, "stringent conditions" mean: (1) hybridization and elution at lower ionic strength and higher temperature, such as 0.2×SSC, 0.1% SDS, 60°C; or (2) adding denaturant during hybridization, such as 50% (v/v) formamide, 0.1% calf serum/0. 1% Ficoll, 42°C, etc.; or (3) hybridization only occurs when the identity between two sequences is at least 90% or more, preferably 95% or more. Also, the polypeptides encoded by the hybridizable polynucleotides have the same biological functions and activities as the mature polypeptides.

The full-length nucleotide sequence of the antibody of the present invention or a fragment thereof generally can be obtained by a PCR amplification method, a recombinant method, or an artificial synthesis method. One possible method is to synthesize the relevant sequence(s), especially when the fragment is short, by artificial synthesis. Typically, long fragments are obtained by first synthesizing a plurality of small fragments and then ligating them together. In addition, the coding sequence of the heavy chain and an expression tag (e.g., 6His) can be fused together to form a fusion protein. The relevant sequence can be obtained in large quantities by a recombinant method after the sequence is obtained, which generally includes the steps of cloning the relevant sequence into a vector, transferring the vector into cells, and isolating and obtaining the relevant sequence from the propagated host cells by conventional methods. The biomolecules (nucleic acid, protein, etc.) to which the present invention relates include biomolecules existed in an isolated form.

At present, the DNA sequence encoding the protein of the present invention (or fragment or derivative thereof) can be obtained completely by chemical synthesis. The DNA sequence can then be introduced into various existing DNA molecules (or e.g., vectors) and cells known in the art. Furthermore, mutations can also be introduced into the protein sequences of the present invention by chemical synthesis.

The present invention also relates to a vector comprising a suitable DNA sequence as described above and a suitable promoter or control sequence. These vectors may be used to transform an appropriate host cell to enable expression of the protein.

The host cell may be a prokaryotic cell, such as a bacterial cell; or lower eukaryotic cells, such as yeast cells; or higher eukaryotic cells, such as mammalian cells. Representative examples include Escherichia coli, Streptomyces; bacterial cells, e.g., of Salmonella typhimurium; fungal cells such as yeast; insect cells, e.g., of Drosophila S2 or Sf 9; animal cells, e.g., of CHO, COS7, 293 cells, etc.

Transformation of a host cell with recombinant DNA may be carried out using conventional techniques well known to those skilled in the art. When the host is prokaryotic, such as E.coli, competent cells, which are capable of uptaking DNA, can be harvested after exponential growth phase and treated by the CaCl₂ approach using procedures well known in the art. Another approach is to use MgCl₂. If desired, transformation can also be carried out by electroporation. When the host is a eukaryote, the following DNA transfection methods may be used: calcium phosphate coprecipitation, conventional mechanical methods such as microinjection, electroporation, liposome packaging, etc.

The obtained transformant can be cultured by a conventional method to express the polypeptide encoded by the gene of the present invention. The medium used in the culture may be selected from various conventional media depending on the host cell used. The culturing is performed under conditions suitable for the growth of the host cell. After the host cells have been grown to an appropriate cell density, the selected promoter is induced by an appropriate method (e.g., temperature shift or chemical induction) and the cells are cultured for an additional period of time.

The recombinant polypeptide in the above method may be expressed intracellularly or on the cell membrane, or secreted into the outside of the cell. If necessary, the physical, chemical and other properties of the recombinant protein can be utilized for isolation and purification of the recombinant protein by various isolation methods. These methods are well known to those skilled in the art. Examples of such methods include, but are not limited to: conventional reconstitution treatment, treatment with a protein precipitant (such as salting-out), centrifugation, cell lysis by osmosis, sonication, ultracentrifugation, molecular sieve chromatography (gel filtration), adsorption chromatography, ion exchange chromatography, High Performance Liquid Chromatography (HPLC), and other various liquid chromatography techniques and combinations thereof.

The antibodies of the present invention may be used alone or in combination or conjugated with detectable labels (for diagnostic purposes), therapeutic agents, PK (protein kinase) modifying moieties or combinations of any of the above.

Detectable labels for diagnostic purposes include, but are not limited to a fluorescent or luminescent label, a radioactive label, an MRI (magnetic resonance imaging) or CT (computed tomography) contrast agent, or an enzyme capable of producing a detectable product.

Therapeutic agents that may be bound or conjugated to the antibodies of the present invention include, but are not limited to: 1. radionuclides; 2. biotoxins; 3. cytokines such as IL-2, etc.; 4. gold nanoparticles/nanorods; 5. viral particles; 6. liposomes; 7. nano magnetic particles; 8. prodrug activating enzymes (e.g., DT-diaphorase (DTD) or biphenyl hydrolase-like protein (BPHL)); 10. chemotherapeutic agents (e.g., cisplatin) or any form of nanoparticles, etc.

### Construction method of the present invention

In the present invention, there is provided a method for construction of a multispecific antibody, comprising the steps of:
(i) constructing a first polynucleotide encoding a first polypeptide having a structure represented by Formula I from N-terminus to C-terminus and a second polynucleotide encoding a second polypeptide having a structure represented by Formula II from N-terminus to C-terminus, respectively,

   A1-L1-B1-L2-CL-L3-A2 (Formula I)

   A3-L4-B2-L5-CH1-L6-A4 (Formula II)

   wherein, A1, A2, A3, and A4 are each independently an antibody or an antigenic fragment thereof targeting a target of interest, and the target antigens targeted by each of A1, A2, A3, and A4 can be the same or different; L1, L2, L3, and L4 are each independently a null or linker element; B1 and B2 are both null, or B1 and B2 are the VL and VH regions, respectively, of an antibody targeting the same target; and a disulfide bond may be formed between the CL region of the first polypeptide and the CH1 region of the second polypeptide, such that the antibody has a heterodimeric form;
(ii) expressing the first polynucleotide and the second polynucleotide to obtain the first polypeptide and the second polypeptide, and dimerizing the first polypeptide and the second polypeptide to form a multispecific antibody with a heterodimeric form. Preferably, the CL region of the first polypeptide has an amino acid sequence as set forth in SEQ ID NO. 9 and the CH1 region of the second polypeptide has an amino acid sequence as set forth in SEQ ID NO. 3, wherein a disulfide bond may be formed between the two regions.

### Pharmaceutical composition

The present invention also provides a composition. Preferably, the composition is a pharmaceutical composition comprising the above antibody or an active fragment thereof or a fusion protein thereof, and a pharmaceutically acceptable carrier. Generally, these substances will be formulated in a non-toxic, inert, and pharmaceutically acceptable aqueous carrier medium, typically having a pH of from about 5 to about 8, preferably a pH of from about 6 to about 8, although the pH will vary depending on the nature of the substances being formulated and the condition being treated. The formulated pharmaceutical compositions may be administered by conventional routes including, but not limited to: intratumoral, intraperitoneal, intravenous, or topical administration.

The pharmaceutical composition of the present invention can be directly used for binding target molecules of interest, and thus can be used for treating the corresponding diseases. In addition, other therapeutic agents may be used simultaneously.

The pharmaceutical composition of the present invention comprises a safe and effective amount (e.g., 0.001-99wt%, preferably 0.01-90wt%, more preferably 0.1-80wt%) of the above-mentioned antibody (or conjugate thereof) of the present invention and a pharmaceutically acceptable carrier or excipient. Such vectors include (but are not limited to): saline, buffer, glucose, water, glycerol, ethanol, and combinations thereof. The pharmaceutical formulation should be compatible with the mode of administration. The pharmaceutical composition of the present invention can be prepared in the form of injection, for example, by a conventional method using physiological saline or an aqueous solution containing glucose and other adjuvants. Pharmaceutical compositions such as injections, solutions are preferably prepared under sterile conditions. The administration amount of active ingredient is a therapeutically effective amount, for example, from about 10 micrograms per kilogram of body weight to about 50 milligrams per kilogram of body weight per day. In addition, the polypeptides of the present invention may also be used with other therapeutic agents.

When using pharmaceutical compositions, a safe and effective amount of the immunoconjugate is administered to a mammal, wherein the safe and effective amount is typically at least about 10 micrograms per kilogram of body weight, and in most cases no more than about 50 mg per kilogram of body weight, preferably the dose is from about 10 micrograms per kilogram of body weight to about 10 mg per kilogram of body weight. Of course, the particular dosage will also take into account such factors as the route of administration, the healthy conditions of the patient, etc., which are within the skill of the skilled physician.

### The main advantages of the present invention include:

1) the bi/multispecific antibody structure of the present invention can simultaneously bind different targets and maintain the binding activity of the original antibody;
2) the bi/multispecific antibody structure of the present invention are effective when the target is a membrane surface receptor or a target in solution;
3) the bi/multispecific antibody structure of the present invention has biological activity against multiple targets;
4) The bi/multispecific antibody structures of the present invention may be linked to a single domain antibody or a normal antibody or an Fc fragment;
5) the bi/multispecific antibody of the present inventin is an antibody or fusion protein constructed by taking CH1-CL dimer as a center, and based on which, the present invention also provides a multispecific antibody comprising an Fc fragment, which can significantly improve the half-life of the protein and simplify the purification process.

The present invention will be further illustrated with reference to the following specific examples. It should be understood that these examples are for illustrative purposes only and are not intended to limit the scope of the present invention. Experimental procedures without specific conditions noted in the following examples are generally carried out following conventional conditions described in, for example Sambrook et. al, Molecular Cloning: A Laboratory Manual (New York: Cold Spring Harbor Laboratory Press, 1989), or according to the manufacturer's recommendations. Unless otherwise indicated, percentages and parts are by weight.

**Table B Summary of sequences of the present invention**

| | | |
|---|---|---|
| Peptide #1 ofBi-70-71 | Amino acid sequence | SEQ ID NO: 1 |
| | Nucleotide sequence | SEQ ID NO: 32 |
| VH chain of anti-PD-1 antibody | Amino acid sequence | SEQ ID NO: 2 |
| | Nucleotide sequence | SEQ ID NO: 33 |
| CH1 region of human IgG1 | Amino acid sequence | SEQ ID NO: 3 |
| | Nucleotide sequence | SEQ ID NO: 34 |
| Linker sequence | Amino acid sequence | SEQ ID NO: 4 |
| Anti-human serum albumin nanobody | Amino acid sequence | SEQ ID NO: 5 |
| | Nucleotide sequence | SEQ ID NO: 35 |
| Anti-TIGIT nanobody | Amino acid sequence | SEQ ID NO: 6 |
| | Nucleotide sequence | SEQ ID NO: 36 |
| Peptide #2 of Bi-70-71 | Amino acid sequence | SEQ ID NO: 7 |
| | Nucleotide sequence | SEQ ID NO: 37 |
| VL chain of anti-PD-1 antibody | Amino acid sequence | SEQ ID NO: 8 |
| | Nucleotide sequence | SEQ ID NO: 38 |
| Human kappa light chain constant region CL | Amino acid sequence | SEQ ID NO: 9 |
| | Nucleotide sequence | SEQ ID NO: 39 |
| Peptide #1 ofBi-72-73 | Amino acid sequence | SEQ ID NO: 10 |
| | Nucleotide sequence | SEQ ID NO: 40 |
| Peptide #2 of Bi-72-73 | Amino acid sequence | SEQ ID NO: 11 |
| | Nucleotide sequence | SEQ ID NO: 41 |
| Peptide #1 ofBi-74-76 | Amino acid sequence | SEQ ID NO: 12 |
| | Nucleotide sequence | SEQ ID NO: 42 |
| VH chain of anti-VEGF antibody | Amino acid sequence | SEQ ID NO: 13 |
| | Nucleotide sequence | SEQ ID NO: 43 |
| Anti-PD-L1 nanobody | Amino acid sequence | SEQ ID NO: 14 |
| | Nucleotide sequence | SEQ ID NO: 44 |
| Peptide #2 of Bi-74-76 | Amino acid sequence | SEQ ID NO: 15 |
| | Nucleotide sequence | SEQ ID NO: 45 |
| VL chain of anti-VEGF antibody | Amino acid sequence | SEQ ID NO: 16 |
| | Nucleotide sequence | SEQ ID NO: 46 |
| Peptide #1 of Bi-78-79, Bi-79-83, Bi-79-86 | Amino acid sequence | SEQ ID NO: 17 |
| | Nucleotide sequence | SEQ ID NO: 47 |
| Peptide #2 of Bi-78-79, Bi-79-80 | Amino acid sequence | SEQ ID NO: 18 |
| | Nucleotide sequence | SEQ ID NO: 48 |
| Anti-PD-L2 nanobody | Amino acid sequence | SEQ ID NO: 19 |
| | Nucleotide sequence | SEQ ID NO: 49 |
| Peptide #1 ofBi-78-80 | Amino acid sequence | SEQ ID NO: 20 |
| | Nucleotide sequence | SEQ ID NO: 50 |
| Linker sequence of Bi-78-80 | Amino acid sequence | SEQ ID NO: 21 |
| Peptide #1 of Bi-81-82 | Amino acid sequence | SEQ ID NO: 22 |
| | Nucleotide sequence | SEQ ID NO: 51 |
| Peptide #2 of Bi-81-82 | Amino acid sequence | SEQ ID NO: 23 |
| | Nucleotide sequence | SEQ ID NO: 52 |
| Peptide #2 of Bi-79-83 | Amino acid sequence | SEQ ID NO: 24 |
| | Nucleotide sequence | SEQ ID NO: 53 |
| Peptide #2 of Bi-79-86 | Amino acid sequence | SEQ ID NO: 25 |
| | Nucleotide sequence | SEQ ID NO: 54 |
| Anti-41BB nanobody | Amino acid sequence | SEQ ID NO: 26 |
| | Nucleotide sequence | SEQ ID NO: 55 |
| Peptide #1 of Bi-203-204 | Amino acid sequence | SEQ ID NO: 27 |
| | Nucleotide sequence | SEQ ID NO: 56 |
| Anti-PD-L1 nanobody | Amino acid sequence | SEQ ID NO: 28 |
| | Nucleotide sequence | SEQ ID NO: 57 |
| LALA mutant Fc of human IgG1 | Amino acid sequence | SEQ ID NO: 29 |
| | Nucleotide sequence | SEQ ID NO:58 |
| Peptide #2 of Bi-203-204 | Amino acid sequence | SEQ ID NO: 30 |
| | Nucleotide sequence | SEQ ID NO: 59 |
| Anti-PD-L2 nanobody | Amino acid sequence | SEQ ID NO: 31 |
| | Nucleotide sequence | SEQ ID NO: 60 |

### Example 1: Anti-PD-1/TIGIT/human serum albumin trispecific antibody

### 1.1 Construction of anti-PD-1/TIGIT/human serum albumin trispecific antibody

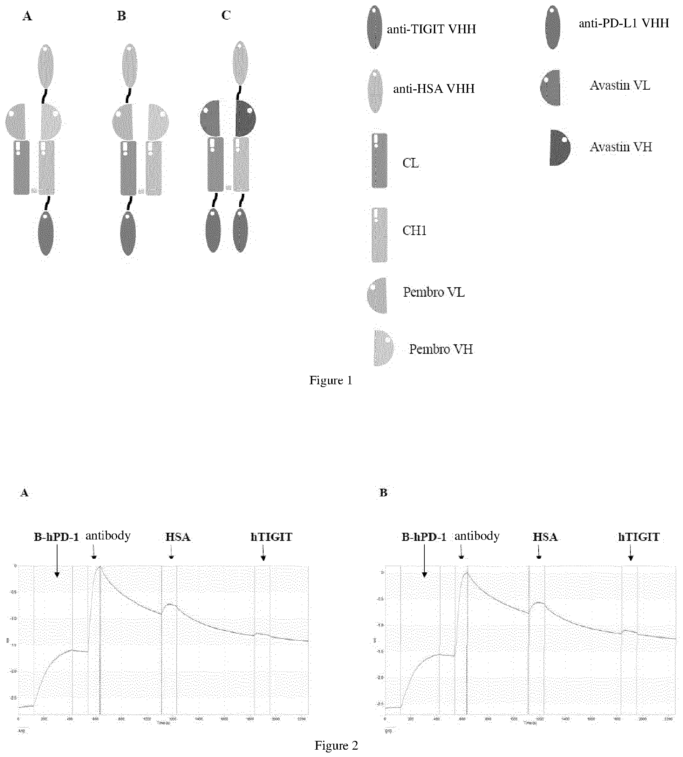

To demonstrate the M-Body technology, in this example, two anti-PD-1/TIGIT/human serum albumin trispecific antibodies were constructed:
Bi-70-71 consisting of 2 polypeptide chains had a structure as shown in FIG. 1A, wherein peptide chain #1 had an amino acid sequence as set forth in SEQ ID NO. 1, which contained a VH amino acid sequence (SEQ ID NO:2) derived from the anti-PD-1 antibody Pembrolizumab (Patent No. US8354509), the C-terminus of the VH amino acid sequence being directly linked to a CH1 amino acid sequence (SEQ ID NO:3) derived from human IgG1; and the peptide chain #1 was obtained by linking the C-terminus of nanobody ALB8 (Patent No.: WO2004/041865) having SEQ ID NO:5 anti-human serum albumin to the N-terminus of a heavy chain variable region of Pembrolizumab via a flexible peptide of 11 amino acid residues (GGGGSGGGGSG) (SEQ ID NO: 4), and linking the N-terminus of anti-TIGIT nanobody E-Ye-11 (SEQ ID NO:6) to the C-terminus of CH1 via a flexible peptide of 11 amino acid residues (GGGGSGGGGSG); peptide chain #2 had an amino acid sequence as set forth in SEQ ID NO:7, which contained a VL amino acid sequence (SEQ ID NO: 8) derived from the anti-PD-1 antibody Pembrolizumab; and the peptide chain #2 was obtained by directly linking the C-terminus of the VL amino acid sequence to a human kappa light chain constant region (CL) amino acid sequence (SEQ ID NO:9).

Bi-72-73 also consisting of 2 polypeptide chains had a structure as shown in FIG. 1B, wherein peptide chain #1 had an amino acid sequence as set forth in SEQ ID NO: 10, which contained a VH amino acid sequence derived from the anti-PD-1 antibody Pembrolizumab (SEQ ID NO:2), the C-terminus of the VH amino acid sequence being directly linked to a CH1 amino acid sequence derived from human IgG1 (SEQ ID NO: 3); peptide chain #2 had an amino acid sequence as set forth in SEQ ID NO: 11, which contained a VL amino acid sequence (SEQ ID NO: 8) derived from the anti-PD-1 antibody Pembrolizumab, the C-terminus of the VL amino acid sequence being directly linked to the human kappa light chain constant region (CL) amino acid sequence (SEQ ID NO: 9); and the peptide chain #2 was obtained by linking the C-terminus of the anti-human serum albumin nanobody ALB8 (SEQ ID NO:5) to the N-terminus of the light chain variable region of Pembrolizumab via a flexible peptide of 11 amino acid residues (GGGGSGGGGSG) (SEQ ID NO:4), and linking the N-terminus of the anti- TIGIT nanobody E-Ye-11 (SEQ ID NO:6) to the C-terminus of CL via a flexible peptide of 11 amino acid residues GGGGSGGGGSG (SEQ ID NO:4).

### 1.2 Expression and purification of anti-PD-1/TIGIT/human serum albumin trispecific antibody

In this example, the nucleotide sequences encoding each of the 2 chains of the anti-PD-1/TIGIT/human serum albumin trispecific antibodies Bi-70-71 and Bi-72-73 constructed in Example 1.1 were ligated to a commercially available eukaryotic expression vector pCDNA3.1(+) via a multiple cloning site and expressed and purified in eukaryotic cells to obtain the trispecific antibodies Bi-70-71 and Bi-72-73, with the specific procedure as follows.

### Construction of antibody gene into pCDNA3.1 expression vector

The gene sequences encoding each of the 2 strands of Bi-70-71 and Bi-72-73 were synthesized by GENEWIZ, Inc., then ligated to the linearized pCDNA3.1 vector with the homologous recombinase (purchased from Vazyme) and EcoR I/Not I double digestion, following the procedures in commercial instructions. The homologous recombination products were transformed into Top10 competent cells, plated with ampicillin resistant plates, cultured overnight at 37° C, and single clones were picked for sequencing.

### Protein expression and purification

The plasmid was transferred into Expi-CHO cells using ExpiCHO^{™} Expression System Kit (Thermo) by the transfection method according to the commercial instructions, and the supernatant was collected after 5 days of cell culture and purified using a KappaSelect (GE) affinity chromotography column. The specific method was as follows: the sample was filtered by syringe through a 0.2 µm sterile needle filter PES; the chromotography column was equilibrated with 5 column volumes of equilibration buffer (20 mM PB + 0.15M NaCl, pH 7.4) until the effluent conductance and pH remained unchanged; the flow rate was 0.5 ml/min. And after the sample loading was finished, the chromotography column was continuously washed by the balance buffer until the penetration was complete and the UV value was not reduced any more. After eluting with elution buffer (0.1M glycine-HCl, pH 3.0), and the elute was collected. Immediately after elution, the collected antibody solution should be neutralized with an alkaline buffer (e.g., 1M Tris/HCl, pH 8.0) to a pH at which the antibody was stable.

The purity of the protein was determined by HPLC. HPLC method was as follows: mobile phase: 150 mM Na₂HPO₄•12H₂O, pH 7.0. Chromatographic conditions were as follows: detection wavelength: 280 nm, column temperature: 25° C, flow rate: 0.35 ml/min, detection time: 20 min, Zenix-C SEC-300 column (SEPAX 4.6×300 mm, 3 µm). SEC results showed that the bispecific antibody Bi-70-71 was 99.14% pure and Bi-72-73 was 98.27% pure.

### 1.3 Determination of antigen co-binding capacity of anti-PD-1/TIGIT/human serum albumin trispecific antibody

Whether the two exemplary anti-PD-1/TIGIT/human serum albumin trispecific antibodies Bi-70-71 and Bi-72-73 of the present invention described above can bind to PD-1, TIGIT, and human serum albumin simultaneously was determined by kinetic binding assays using the Octet system (manufactured by ForteBio Inc.). Half an hour before the start of the experiment, the SA sensor (Pall) was equilibrated at room temperature by soaking in SD buffer (PBS 1 ×, BSA 0.1%, tween 20 0.05%). To the wells of a 96-well black polystyrene half-microplate (Greiner) were added 100 µL of SD buffer as a blank control (for background subtraction), 100 µL of 100 nM purified bispecific antibodies Bi-70-71 and Bi-72-73, and 100 µL solution of biotinylated-labeled human PD-1 (100 nM) (ACROBiosystems), human TIGIT (100 nM) (ACROBiosystems), and human serum albumin (ACROBiosystems) as antigens diluted in SD buffer, respectively. The SA sensor was immersed in a well containing a biotinylated-labeled human PD-1 solution for 60 s at room temperature and then loaded. The sensor was then washed in SD buffer to reach baseline and then immersed in a well containing 100 µL antibody solution to monitor the association of the antibody with the antigen, followed by transferring the sensor to a well containing 100 µL SD buffer to monitor the antibody dissociation; then the sensor was transferred to a well containing 100 nM human TIGIT solution to detect the binding of the antibody to the human TIGIT, then the sensor was transferred to a well containing 100 µL SD buffer to monitor the antigen dissociation; the sensor was then transferred to a well containing 100 nM human serum albumin solution to detect the binding of the antibody to the human serum albumin, followed by transferring the sensor to a well containing 100 µl SD buffer to monitor the antigen dissociation. The rotation speed was 1000 rpm and the temperature was 30° C.

In the assay experiment by the method as described above, the anti-PD-1/TIGIT/human serum albumin trispecific antibodies Bi-70-71 (as shown in FIG. 2A) and Bi-72-73 (as shown in FIG. 2B) of the present invention can simultaneously bind to human PD-1, human TIGIT, and human serum albumin protein.

### 1.4 Determination of the binding capacity of the antigen of anti-PD-1/TIGIT/human serum albumin trispecific antibody

CHO cells overexpressing human PD-L1 or human TIGIT (CHO-hPD-L1 cells, CHO-hTIGIIT cells) were generated by transfecting pCHO1.0 vectors (purchased from Invitrogen) with human PD-1 or human TIGIT cDNA (purchased from Nano Biological) cloned into MCS. The amplified CHO-hPD-L1/CHO-hTIGIIT cells were adjusted to a cell density of 2×10⁶ cells/ml, and 100 µL of the cells was added to each well of a 96-well flow plate, followed by centrifugation for later use. The purified trispecific antibody was diluted by 3-fold serial dilutions with PBS(starting at 400nM and 12 concentrations in total), 100 µL of the diluted sample was added to each well of the 96-well flow plate containing cells, and the plate was incubated for 30 min at 4°C and washed twice with PBS. To each well of the purified antibody sample wells was added 100 µL of mouse anti-human IgG-Fab (PE) (purchased from Abcam) diluted with PBS, and the plate was incubated at 4°C for 30 min and washed twice with PBS. 100 µL PBS was added per well to resuspend the cells, which were detected on a CytoFlex (Bechman) flow cytometer to calculate the corresponding MFI.

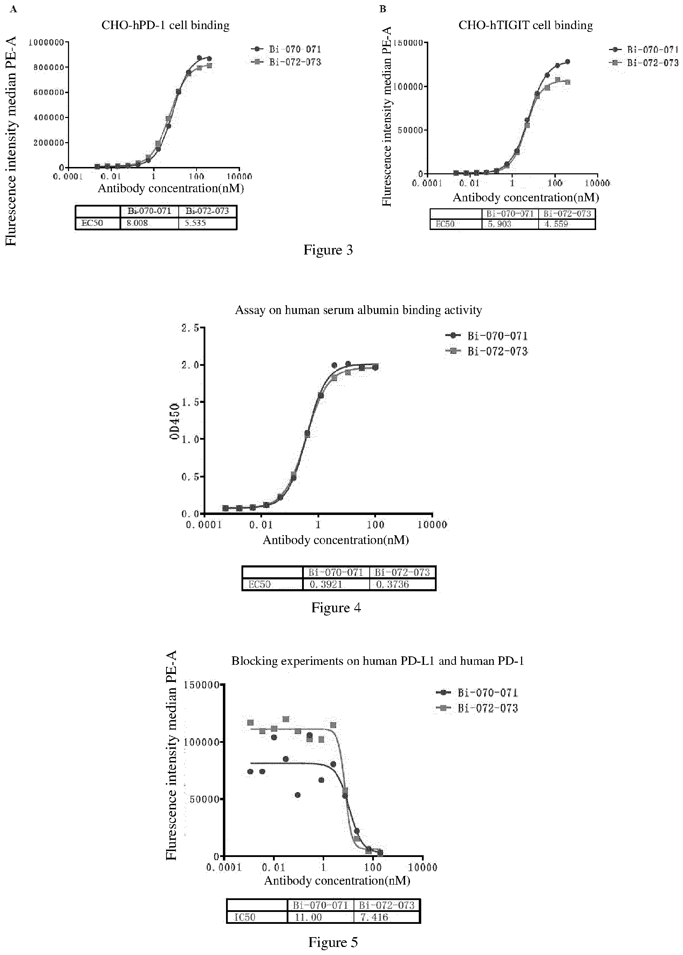

In the assay experiment by the method as described above, as shown in FIG. 3, the results showed that the anti-PD-1/TIGIT/human serum albumin trispecific antibody of the present invention had binding activity to both CHO-hPD-1 cell and CHO-hTIGIIT cell.

### 1.5 Binding of anti-PD-1/TIGIT/human serum albumin trispecific antibody to human serum albumin at an ELISA level

Human serum albumin (ACROBiosystems) was diluted with an ELISA coating solution, then added to ELISA plates, and coated overnight at 4°C. The coating solution was discarded, and 250 µL of PBST was added per well for washing 3 times, and the ELISA plate was blocked with 5% BSA for 1 h at room temperature for later use. The purified and control antibodies were diluted in a gradient, then added to the blocked ELISA plate, and the plate was incubated for 2 h at room temperature and washed with PBST 3 times; to the purified antibody sample wells was added goat anti-human Fab-HRP (Abcam) , and the plate was incubated for 1 h at room temperature and washed with PBST 3 times, followed by adding an ELISA color development solution, standing for 3 min at room temperature, and adding an ELISA stop solution to read the value of absorbance at 450 nm.

In the assay experiment by the method as described above, as shown in FIG. 4, the experimental results showed the binding of anti-PD-1/TIGIT/human serum albumin trispecific antibody of the present invention to human serum albumin at an ELISA level.

### 1.6 Blocking of the binding of human PD-L1 to human PD-1 by anti-PD-1/TIGIT/human serum albumin trispecific antibody

CHO-hPD-1 cells were adjusted to a cell density of 2×10⁶ cells/mL, 100 µL of the cell was added to each well of a 96-well flow plate, followed by centrifugation for later use. The purified and control antibody samples were diluted by 3-fold serial dilutions with PBS(starting at 400nM and 12 concentrations in total), and 60 µL of the diluted sample was added to each well of a 96-well sample dilution plate, while 60 µL of biotinylated-labeled human PD-L1 protein (purchased from ACROBiosystems) was added per well at a final concentration of 500 ng/mL, and the mixture was incubated with the sample for 30 min at 4°C. 100 µL of the co-incubated samples were added to each well of the above 96-well flow plate containing cells, and the plate was incubated at 4°C for 30 min and washed twice with PBS. 100 µL well of Streptavidin, R-phytoerythrin Conjugate (available from Thermo fisher) diluted 100-fold with PBS was added, and the plate was incubated at 4°C for 30 min and washed twice with PBS. 100 µL of PBS was added per well to resuspend the cells, which were detected on a CytoFlex (Bechman) flow cytometer to calculate the corresponding MFI.

In the assay experiment by the method as described above, as shown in FIG. 5, the experiment results showed that the anti-PD-1/TIGIT/human serum albumin trispecific antibody of the present invention can block the binding of PD-L1 to PD-1.

### 1.7 Blocking of the binding of human CD155 to human TIGIT by anti-PD-1/TIGIT/human serum albumin trispecific antibody

CHO-hTIGIT cells were adjusted to a cell density of 2×10⁶ cells/mL, 100 µL of the cell was added to each well of a 96-well flow plate, followed by centrifugation for later use. The purified antibody and control antibody samples were diluted by 3-fold serial dilutions with PBS(starting at 400nM and 12 concentrations in total), and 60 µL of the diluted sample was added to each well of the 96-well sample dilution plate, while 60 µL of human CD155-mFc protein (purchased from ACROBiosystems) was added per well at a final concentration of 2 µg/mL, and the mixture was incubated with the sample for 30 min at 4 °C. 100 µL of the co-incubated samples were added to each well of the above 96-well flow plate containing cells, the plate was plate at 4°C for 30 min, and washed twice with PBS. 100 µL of goat anti-mouse IgGFc-APC (purchased from Biolegend) diluted 100-fold with PBS was added per well, and the plate was incubated at 4°C for 30 min and washed twice with PBS. 100 µL of PBS was added per well to resuspend the cells, which were detected on a CytoFlex (Bechman) flow cytometer to calculate the corresponding MFI.

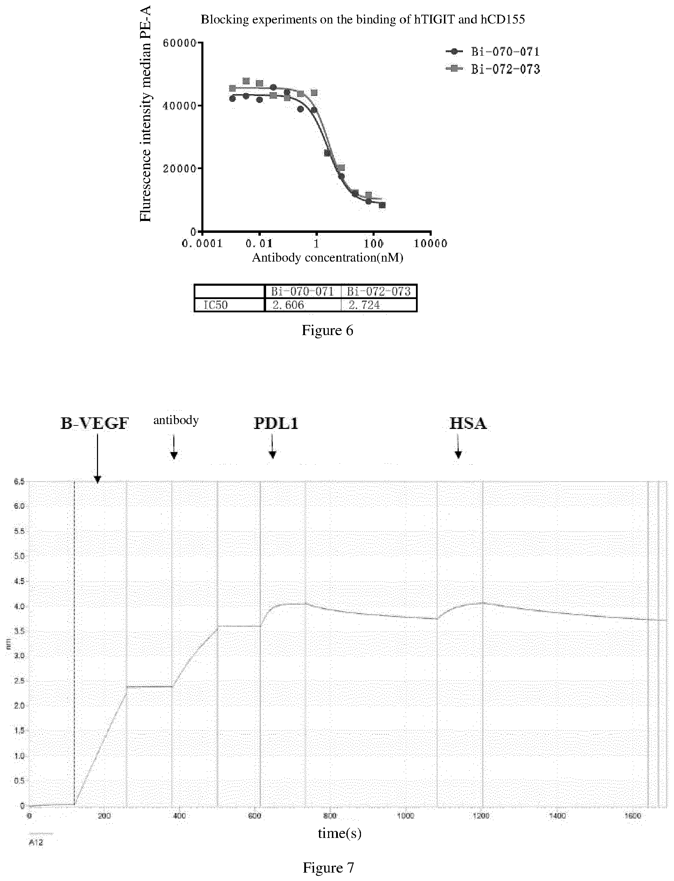

In the assay experiment by the method as described above, as shown in FIG. 6, the experiment results showed that the anti-PD-1/TIGIT/human serum albumin trispecific antibodies of the present invention can block the binding of CD155 to TIGIT.

In summary, the binding activity of the parent antibody may be effectively maintained by linking VH and VL domains over the CH1-CL domain while linking one or more nanobody domains to form a bispecific or multispecific antibody. The VH-VL combination used in this example derived from Pembrolizumab was a domain binding to the cell surface antigen human PD-1.

### Example 2: Anti-VEGF/PD-L1/human serum albumin trispecific antibody

### 2.1. Construction of anti-VEGF/PD-L1/human serum albumin trispecific antibody

To confirm whether M-body was applicable when the linked VH-VL domain was targeted to free antigens in the blood, in this example, one anti-VEGF/PD-L1/human serum albumin trispecific antibody consisting of 2 polypeptide chains, designated as Bi-74-76, was constructed, with the schematic structure shown in FIG. 1C, wherein peptide chain #1 had an amino acid sequence as set forth in SEQ ID NO: 12, which contained a VH amino acid sequence (SEQ ID NO: 13) derived from the anti-VEGF antibody Bevacizumab (Patent No: WO1998045332), the C-terminus of the VH amino acid sequence being directly linked to a CH1 amino acid sequence (SEQ ID NO:3) derived from human IgG1; and the peptide chain #1 was obtained by linking the C-terminus of the anti-human serum albumin nanobody ALB8 (SEQ ID NO:5) to the N-terminus of the Bevacizumab heavy chain variable region via a flexible peptide of 11 amino acid residues (GGGGSGGGGSG) (SEQ ID NO:4), and linking the N-terminus of the anti-human PD-L1 nanobody C-Ye-8-5 (Patent Application No. 2019108631090) (SEQ ID NO:14) to the C-terminus of CH1 via a flexible peptide of 11 amino acid residues (GGGGSGGGGSG) (SEQ ID NO:4); peptide chain #2 had an amino acid sequence as set forth in SEQ ID NO. 15, which contained a VL amino acid sequence (SEQ ID NO: 16) derived from anti-VEGF antibody Bevacizumab, the C-terminus of the VL amino acid sequence being directly linked to an amino acid sequence (SEQ ID NO:9) derived from the human kappa light chain constant region (CL); and the peptide chain #2 was obtained by linking the N-terminus of anti-human PD-L1 nanobody C-Ye-8-5(SEQ ID NO: 14) to the C-terminus of CL via a flexible peptide of 11 amino acid residues (GGGGSGGGGSG) (SEQ ID NO:4).

### 2.2 Expression and purification of anti-VEGF/PD-L1/human serum albumin trispecific antibody

In this example, two nucleotide sequences encoding the anti-VEGF/PD-L1/human serum albumin trispecific antibody Bi-74-76 constructed in Example 2.1 were all ligated into a commercially available eukaryotic expression vector pCDNA3.1(+) via a multiple cloning site, and expressed and purified in eukaryotic cells to obtain the trispecific antibody Bi-74-76. Expression plasmid construction, cell transfection, protein purification and HPLC purity detection methods were the same as Example 1.2. SEC results showed that the bispecific antibody Bi-74-76 was 95.89% pure.

### 2.3 Determination of the antigen co-binding capacity of anti-VEGF/PD-L1/human serum albumin trispecific antibody

Whether the two exemplary anti-VEGF/PD-L1/human serum albumin trispecific antibodies Bi-74-76 of the present invention described above can bind to human PD-L1, VEGF, and human serum albumin simultaneously was determined by a kinetic binding assay using the Octet system (ForteBio Inc.). Half an hour before the start of the experiment, the SA sensor (Pall) was equilibrated at room temperature by soaking in SD buffer (PBS 1×, BSA 0.1%, tween 20 0.05%). To the wells of a 96-well black polystyrene half-well microplate (Greiner) were added 100 µL of SD buffer as a blank control (for background subtraction), 100µL of 100 nM purified trispecific antibody Bi-74-76, 100µL of biotinylated-labeled human VEGF (100 nM) (ACROBiosystems) diluted in SD buffer as antigen, human PD-L1 (100 nM) (ACROBiosystems), and a solution of human serum albumin (ACROBiosystems), respectively. The SA sensor was immersed in a well containing biotinylated-labeled VEGF solution for 60 s at room temperature and then loaded. The sensor was then washed in SD buffer to reach baseline and then immersed in a well containing 100 µL antibody solution to monitor the association of the antibody with the antigen, followed by transferring the sensor to a well containing 100 µL SD buffer to monitor the antibody dissociation; the sensor was then transferred to a well containing 100 nM human PD-L1 solution to detect the binding of the antibody to human PD-L1, followed by transferring the sensor to a well containing 100 µL SD buffer to monitor antigen dissociation; the sensor was then transferred to a well containing 100 nM human serum albumin solution to detect the binding of the antibody to the human serum albumin, followed by transferring the sensor to a well containing 100 µ l SD bufferto monitor the antigen dissociation. The rotation speed was 1000 rpm and the temperature was 30°C.

In the assay experiment by the method as described above, as shown in FIG. 7, the experiment results showed that the anti-VEGF/PD-L1/human serum albumin trispecific antibody Bi-74-76 can bind to human PD-L1, human VEGF, and human serum albumin at the same time.

### 2.4 Determination of the antigen binding capacity of the anti-VEGF/PD-L1/human serum albumin trispecific antibody

CHO cells overexpressing human PD-L1 (CHO-hPD-L1, CHO-hTIGIT cells) were generated by transfecting pCHO1.0 vector (purchased from Invitrogen) with human PD-L1 cDNA (purchased from Nano Biological) cloned into MCS. Binding activities of the anti-VEGF/PD-L1/human serum albumin trispecific antibody to CHO-hPD-L1 cell were detected as in Example 1.4.

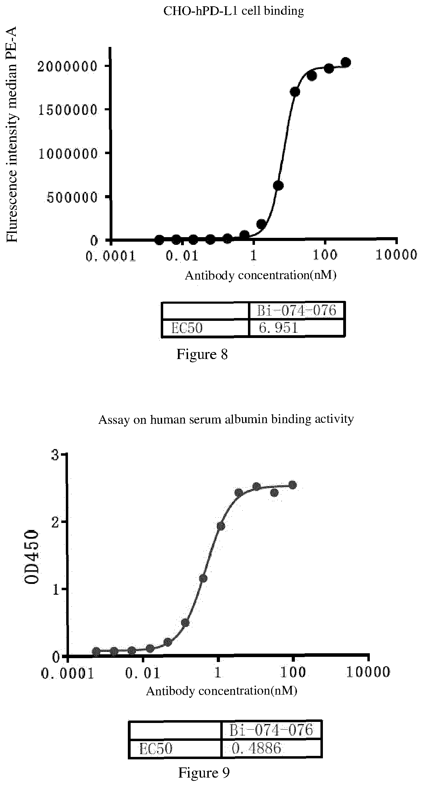

In the assay experiments by the method as described above, as shown in FIG. 8, the results showed that the anti-VEGF/PD-L1/human serum albumin trispecific antibody of the present invention can bind to CHO-hPD-L1 cells.

### 2.5 Determination of the binding of anti-VEGF/PD-L1/human serum albumin trispecific antibody to human serum albumin at an ELISA level

The binding capacity of the anti-VEGF/PD-L1/human serum albumin trispecific antibody to the human serum albumin was detected by a method of ELISA reaction in this experiment, and the experimental method was the same as the Example 1.5. In the assay experiments by the method as described above, the purified antibody of the present invention was able to bind to human serum albumin at an ELISA level (see FIG. 9).

### 2.6 Binding of anti-VEGF/PD-L1/human serum albumin trispecific antibody to human VEGF at an ELISA level

Human VEGF (ACROBiosystems) protein was diluted with an ELISA coating solution, then added to ELISA plates, and coated overnight at 4°C. The coating solution was discarded, and 250 µL of PBST was added per well washing 3 times, and the ELISA plate was blocked with 5% BSAfor 1 h at room temperature. The purified antibody Bi-074-076 antibody was diluted in a gradient, then added to the blocked ELISA plate, and the plate was incubated at room temperature for 2 h and washed with PBST 3 times; to the purified antibody sample wells was added goat anti-human Fab-HRP (Abcam), to the control antibody sample wells was added goat anti-human Fc-HRP (Abcam), and the plate was incubated for 1 h at room temperature and washed with PBST 3 times, followed by adding an ELISA developing solution, standing for 3 minutes at room temperature, and adding an ELISA stopping solution to read the value of absorbance at 450 nm.

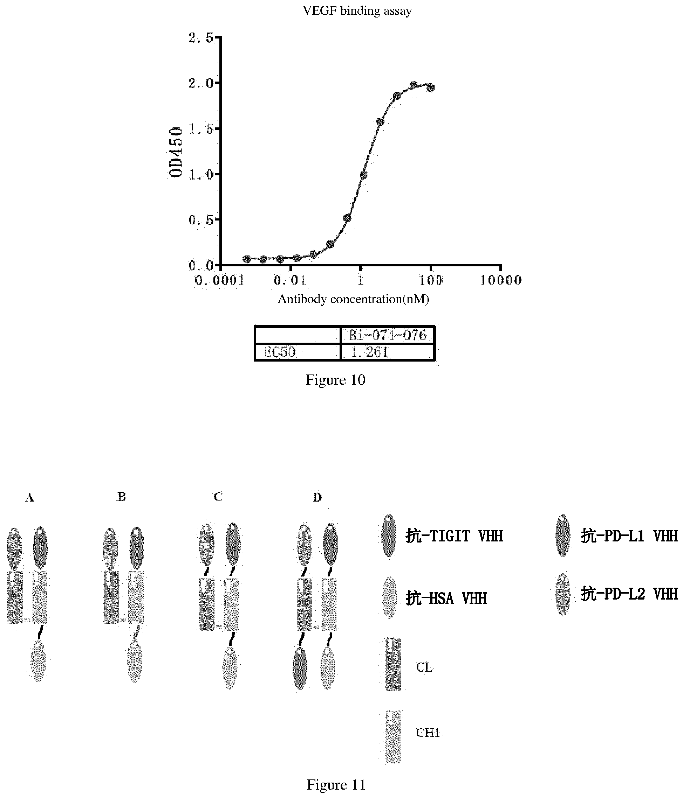

In the assay experiments by the method as described above, the purified antibody Bi-74-76 of the present invention was able to bind to human VEGF protein at an ELISA level (see FIG. 10).

In summary, the binding activity of the parent antibody can be effectively maintained by linking VH and VL domains over the CH1-CL structure, while linking one or more nanobody domains to form a bispecific or multispecific antibody. The VH-VL combination used in this example derived from Bevacizumab was a domain binding to the free antigen VEGF in blood.

### Example 3: Anti-PD-L1/PD-L2/human serum albumin trispecific antibody or anti-PD-L1/PD-L2/TIGIT/human serum albumin tetraspecific antibody

### 3.1 Construction of anti-PD-L1/PD-L2/human serum albumin trispecific antibody or anti-PD-L1/PD-L2/TIGIT/human serum albumin tetraspecific antibody

In order to confirm whether the M-body was applicable when binding two nano-antibodies targeting different targets in the same cell, the inventor constructed a group of anti-PD-L1/PD-L2/human serum albumin trispecific antibodies or anti-PD-L1/PD-L2/TIGIT/human serum albumin tetraspecific antibodies.

In this example, three anti-PD-L1/PD-L2/human serum albumin trispecific antibodies were constructed:
Bi-78-79 consisting of 2 polypeptide chains had a schematic structure shown in FIG. 11A, wherein peptide chain #1 had an amino acid sequence as set forth in SEQ ID NO: 17, which contained anti-PD-L1 nanobody C-Ye-18-5 (SEQ ID NO: 14), the C-terminus of the nanobody amino acid sequence being directly linked to a CH1 amino acid sequence (SEQ ID NO:3) derived from human IgG1; and the peptide chain #1 was obtained by linking the C-terminus of the anti-human serum albumin nanobody ALB8 (SEQ ID NO:5) to the C-terminus of CH1 region via a flexible peptide of 11 amino acid residues (GGGGSGGGGSG) (SEQ ID NO:4); peptide chain #2 had an amino acid sequence as set forth in SEQ ID NO: 18, which contained the anti-PD-L2 nanobody DYe-22 amino acid sequence (SEQ ID NO: 19), and the peptide chain #2 was obtained by directly linking the C-terminus of the nanobody amino acid sequence to the human kappa light chain constant region (CL) amino acid sequence (SEQ ID NO:9).

Bi-78-80 consisting of 2 polypeptide chains had a schematic structure shown in FIG. 1A, wherein peptide chain #1 had an amino acid sequence as set forth in SEQ ID NO:20, which contained anti-PD-L1 nanobody SEQ ID NO:2, the C-terminus of the nanobody amino acid sequence being directly linked to a CH1 amino acid sequence SEQ ID NO: 6 derived from human IgG1; which contained anti-PD-L1nanobody C-Ye-18-5 (SEQ ID NO:14) , the C-terminus of the nanobody amino acid sequence being directly linked to a CH1 amino acid sequence (SEQ ID NO:3) derived from human IgG1; and the peptide chain #1 was obtained by linking the C-terminus of the anti-human serum albumin nanobody ALB8 (SEQ ID NO:5) to the C-terminus of a CH1 region via a flexible peptide of 5 amino acid residues (DKTHT) (SEQ ID NO: 21); peptide chain #2 had an amino acid sequence as set forth in SEQ ID NO 18.

Bi-81-82 consisting of 2 polypeptide chains had a schematic structure shown in FIG. 1A, wherein peptide chain #1 had an amino acid sequence as set forth in SEQ ID NO:22, which contained anti-PD-L1 nanobody C-Ye-18-5 (SEQ ID NO:14), the C-terminus of the nanobody amino acid sequence being linked to a CH1 amino acid sequence (SEQ ID NO:3) derived from human IgG1 via a flexible peptide chain of 11 amino acids (GGGGSGGGGSG) (SEQ ID NO:4); and the peptide chain #1 was obtained by linking the C-terminus of anti-human serum albumin nanobody ALB8 (SEQ ID NO:5) to the C-terminus of a CH1 region via a flexible peptide of 11 amino acid residues (GGGGSGGGGSG) (SEQ ID NO:4); peptide chain #2 had an amino acid sequence as set forth in SEQ ID NO:23, which contained anti-PD-L2 nanobody D-Ye-22 amino acid sequence (SEQ ID NO: 19), and the peptide chain #2 was obtained by linking the C-terminus of the nanobody amino acid sequence to a human kappa light chain constant region (CL) amino acid sequence (SEQ ID NO:9) via a flexible peptide chain of 11 amino acids (GGGGSGGGGSG) (SEQ ID NO:4).

In this example, one anti-PD-L1/PD-L2/TIGIT/human serum albumin tetraspecific antibody consisting of 2 polypeptide chains, designated as Bi-79-83, was constructed, with the schematic structure as shown in FIG. 1A, wherein peptide chain #1 had an amino acid sequence as set forth in SEQ ID NO: 17; peptide chain #2 had an amino acid sequence as set forth in SEQ ID NO:24, which contained the anti-PD-L2 nanobody DYe-22 amino acid sequence (SEQ ID NO:19), at the C-terminus of the nanobody amino acid sequence being directly linked to a human kappa light chain constant region (CL) amino acid sequence (SEQ ID NO: 9); and the peptide chain #2 was obtained by linking the N-terminus of anti-TIGIT nanobody E-Ye-11 to the C-terminus of CL region via a flexible peptide of 11 amino acid residues (GGGGSGGGGSG) (SEQ ID NO:4).

### 3.2 Expression and purification of PD-L1/PD-L2/human serum albumin trispecific antibody or anti-PD-L1/PD-L2/TIGIT/human serum albumin tetraspecific antibody

In this example, the nucleotide sequences encoding 2 chains of the anti-PD-L1/PD-L2/human serum albumin trispecific antibodies Bi-78-79, Bi-78-80, and Bi-81-82 and the anti-PD-L1/PD-L2/TIGIT/human serum albumin tetraspecific antibody Bi-79-83 constructed in Example 3.1 were ligated into a commercially available eukaryotic expression vector pCDNA3.1(+) via a multiple cloning site for expression and purification in eukaryotic cells. The expression plasmid construction and protein expression and purification method were the same as Example 1.2.

This study examined the purity of the purified product using an SEC assay, which was the same as Example 1.2. The experimental results showed that all 4 multispecific antibodies had higher purity (Bi-78-79: 98.07%; Bi-78-80: 98.62%; Bi-81-82: 96.31%; Bi-79-83: 99.14%).

### 3.3 Determination of the antigen binding capacity of anti-PD-L1/PD-L2/human serum albumin trispecific antibody or anti-PD-L1/PD-L2/TIGIT/human serum albumin tetraspecific antibody

CHO cells overexpressing human PD-L1 or human PD-L2 or human TIGIT (CHO-hPD-L1 cells, CHO-hPD-L2 cells, CHO-hTIGIIT cells) were generated by transfecting pCHO1.0 vectors (purchased from Invitrogen) with human PD-L1 or human PD-L2 or human TIGIT cDNA (purchased from Nano Biological) cloned into MCS. The amplified CHO-hPD-L1/CHO-hPD-L2/CHO-hTIGIIT cells was adjusted to a cell density of 2×10⁶ cells/mL, 100 µL of the cell was added to each well of a 96-well flow plate, followed by centrifugation for later use. The purified trispecific antibody was diluted by 3-fold serial dilutions with PBS(starting at 400nM and 12 concentrations in total), 100 µL of the diluted sample was added to each well of the 96-well flow plate containing cells, and the plate was incubated for 30 min at 4°C and washed twice with PBS. To each well of the purified antibody sample wells was added with 100 µL of mouse anti-human IgG-Fab (PE) (purchased from Abcam) diluted with PBS, and to the control antibody sample wells was added with goat F (ab')2 anti-human IgG-Fc (PE) (purchased from Abcam) diluted with PBS, and the plate was incubated at 4°C for 30 min and washed twice with PBS. 100 µL of PBS was added per well to resuspend the cells, which were detected on a CytoFlex (Bechman) flow cytometer to calculate the corresponding MFI.

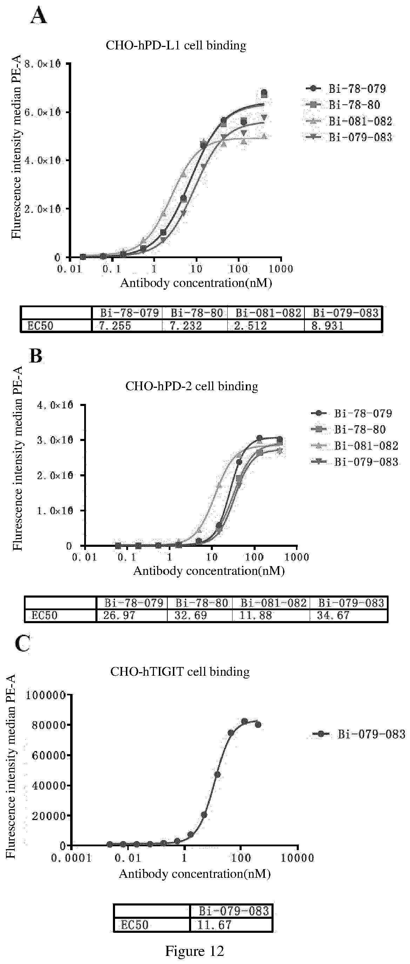

In the assay experiment by the method as described above, as shown in FIG. 12, the results showed that the purified samples of the present invention, Bi-78-79, Bi-78-80 and Bi-81-82 had binding activities to both CHO-hPD-L1 cells and CHO-hPD-L2 cells; the purified samples of the present invention, Bi-79-83 had binding activities to all of CHO-hPD-L1 cells, CHO-hPD-L2 cells and CHO-hTIGIT cells.

### 3.4 Determination of the binding of anti-PD-L1/PD-L2/human serum albumin trispecific antibody or anti-PD-L1/PD-L2/TIGIT/human serum albumin tetraspecific antibody and human serum albumin at an ELISA level

The binding capacity of the anti-PD-L1/PD-L2/human serum albumin trispecific antibody or the anti-PD-L1/PD-L2/TIGIT/human serum albumin tetraspecific antibody and the human serum albumin was detected by a method of ELISA reaction in this experiment, and the experimental method was the same as that in Example 1.3.

In the assay experiment by the method as described above, as shown in IFG. 13, the experimental results showed that all the anti-PD-L1/PD-L2/human serum albumin trispecific antibodies or anti-PD-L1/PD-L2/TIGIT/human serum albumin tetraspecific antibodies of the present invention can bind to human serum albumin at an ELISA level.

### 3.5 Blocking of the binding activity of human PD-L1/PD-L2 to PD-1 by determination of anti-PD-L1/PD-L2/human serum albumin trispecific antibody or anti-PD-L1/PD-L2/TIGIT/human serum albumin tetraspecific antibody

CHO-hPD-1 cells were adjusted to a cell density of 2×10⁶ cells/ml, 100 µL of the cell was added to each well of a 96-well flow plate, followed by centrifugation for later use. The purified antibodies Bi-78-79, Bi-78-80, Bi-81-82, and Bi-79-83 and control antibody samples were diluted by 3-fold serial dilutions with PBS(starting at 400nM and 12 concentrations in total), and 60 µL of the diluted samples were added to each well of the 96-well sample dilution plate, while 60 µL of biotinylated-labeled human PD-L1 protein or biotinylated-labeled human PD-L2 protein (purchased from ACROBiosystems) was added per well at a final concentration of 500 ng/ml, and the mixture was incubated with the samples for 30 min at 4°C. 100 µL of the co-incubated samples were added to each well of the above 96-well flow plate containing cells, and the plate was incubated at 4°C for 30 min and washed twice with PBS. 100 µL of Streptavidin, R-phytoerythrin Conjugate (purchased from Thermo fisher) diluted 100-fold with PBS was added per well, and the plate was incubated at 4°C for 30 min and washed twice with PBS. 100 µL of PBS was added per well to resuspend the cells, which were detected on a CytoFlex (Bechman) flow cytometer to calculate the corresponding MFI.

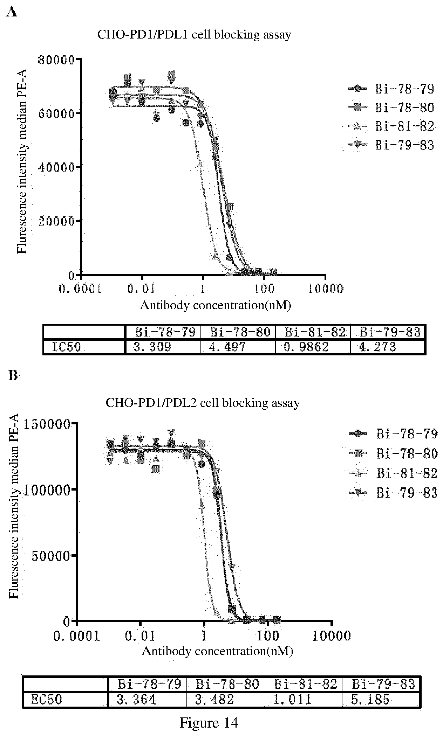

In the assay experiment by the method as described above, as shown in FIG. 14, the experimental results showed that all the anti-PD-L1/PD-L2/human serum albumin trispecific antibodies or anti-PD-L1/PD-L2/TIGIT/human serum albumin tetraspecific antibodies can block the binding of human PD-L1 and human PD-L2 to human PD-1 on the cell surface.

### 3.6 Blocking PDL1/PDL2/PD1/luc signaling pathway experiment by anti-PD-L1/PD-L2/human serum albumin trispecific antibody or anti-PD-L1/PD-L2/TIGIT/human serum albumin tetraspecific antibody

PD-L1 and PD-L2 can be co-expressed on tumor cells or immune cells, and the simultaneous blocking effect of purified antibodies Bi-78-79, Bi-78-80, Bi-81-82, and Bi-79-83 on PD-L1/PD-1 pathway and PD-L2/PD-1 pathway was detected by a method of co-incubation of CHO cells co-expressing human PD-L1 and human PD-L2 and Jurkat cells over-expressing human PD-1 and containing NFAT-Luciferase reporter genes, with the specific procedure as follows.

Functional cells (CHO-K1-PD-L1/PD-L2) co-expressing human PD-L1 and human PD-L2 were adjusted to a density of 5×10⁵ cells/ml, 100 µL of the cell was inoculated to each well of a 96-well cell culture white bottom plate, and cultured overnight in a 5% CO₂ incubator at 37°C. The purified antibody and the control antibody 1640 were diluted in complete medium at a gradient for later use. Jurkat cells overexpressing human PD-1 and containing the NFAT-Luciferase reporter gene (Jurkat-PD-1-NFAT) were adjusted to a cell density of 2.5×10⁵ cells/mL with 1640 complete medium for later use. The white bottom plate was taken out, the culture supernatant was pipetted, adding 40 µL of the diluted sample was added to the white bottom plate per well, 40 µL of Jurkat-PD-1-NFAT effector cell suspension was added simultaneously, and the white bottom plate was placed in a 5% CO₂ incubator at 37°C for adherent culture for 6 h. Bio-Glo (TM) reagent (Promega) was added to each well and the fluorescence signal was read using a multifunctional microplate reader.

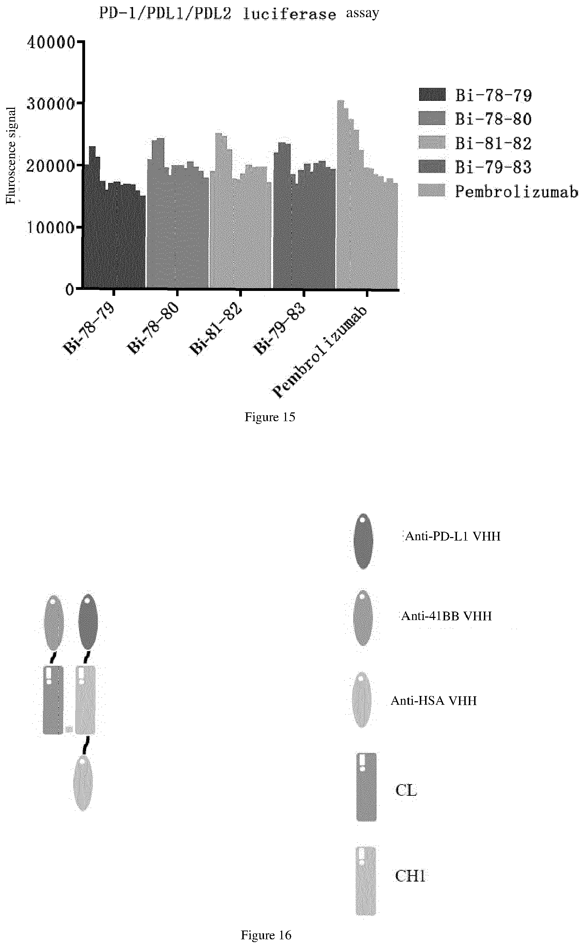

In the assay experiment by the method as described above, as shown in FIG. 15, the experimental results showed that the anti-PD-L1/PD-L2/human serum albumin trispecific antibody or anti-PD-L1/PD-L2/TIGIT/human serum albumin tetraspecific antibody can block PD-L1/PD-1 and PD-L2/PD-1 signaling pathways in vitro at the same time, and the blocking effect was similar to that of anti-PD-1 monoclonal antibody Pembrolizumab.

In summary, the binding activity of the parent antibody can be effectively maintained by linking two different nanobody domains to the N-terminus of the structure of CH1-CL, and linking one nanobody domain to the C-terminus of CH1 to form a trispecific antibody or linking two different nanobody domains to the C-terminus of the structure of CH1-CL to form a tetraspecific antibody. The nanobody can maintain the antigen binding capacity by linking to the N-terminus of CH1 or CL via a flexible peptide chain or directly. The nanobody can maintain the antigen binding capacity by linking to the C-terminus of CH1 via a flexible peptide chain of 11 amino acids (GGGGSGGGGSG) or a short peptide chain of 5 amino acids (DKTHT).

In this example, the combination of anti-PD-L1 and anti-PD-L2 nanobody was used to bind to two antigens on the same cell, and the results indicated that all multispecific antibodies can simultaneously bind PD-L1 to PD-L2 and block the binding of PD-L1/PD-L2 to PD-1, activating downstream signaling pathways.

### Example 4: Anti-PD-L1/41BB/human serum albumin trispecific antibody

### 4.1 Construction of anti-PD-L1/41BB/human serum albumin trispecific antibody

To confirm whether M-body was applicable for linking two nano-antibodies targeting different targets located on different cells, the present inventors constructed an anti-PD-L1/41BB/human serum albumin trispecific antibody designated as Bi-79-86, which contained two different polypeptides, which the schematic structure as shown in FIG. 16. Peptide chain #1 had an amino acid sequence as set forth in SEQ ID NO 17. Peptide chain #2 had an amino acid sequence as set forth in SEQ ID NO:25, which contained an anti-41BB nanobody amino acid sequence (SEQ ID NO:26 Patent Number), and the peptide chain #2 was obtained by directly linking the C-terminus of the nanobody amino acid sequence to a human kappa light chain constant region (CL) amino acid sequence SEQ ID NO:9.

### 4.2 Expression and purification of anti-PD-L1/41BB/human serum albumin trispecific antibody

In this example, the nucleotide sequences encoding the 2 chains of the anti-PD-L1/41BB/human serum albumin trispecific antibody Bi-79-86 constructed in Example 4.1 were all ligated into a commercially available eukaryotic expression vector pCDNA3.1(+) via a multiple cloning site and expressed and purified in eukaryotic cells. The expression plasmid construction and protein expression and purification method were the same as Example 1.2.

This study examined the purity of the purified product using an SEC assay, which was the same as Example 1.2. The experimental result showed that the anti-PD-L1/41BB/human serum albumin trispecific antibody obtained in the research was 95.08% pure.

### 4.3 Determination of the antigen binding capacity of anti-PD-L1/41BB/human serum albumin trispecific antibody

CHO cells overexpressing human PD-L1 or human 41BB (CHO-hPD-L1 cells, CHO-41BB cells) were generated by transfecting pCHO1.0 vectors (purchased from Invitrogen) with human PD-L1 or human 41BB cDNA (purchased from Nano Biological) cloned into MCS. The amplified CHO-hPD-L1/CHO-41BB cells was adjusted to a cell density of 2×10⁶ cells/mL, 100 µ of the cell was added to each well of a 96-well flow plate, followed by centrifugation for later use. The purified trispecific antibody was diluted by 3-fold serial dilutions with PBS(starting at 400nM and 12 concentrations in total), 100 µL of the diluted sample was added to each well of the 96-well flow plate containing cells, and the plate was incubated for 30 min at 4°C and washed twice with PBS. To each well of the purified antibody sample wells was added with 100 µL of mouse anti-human IgG-Fab (PE) (purchased from Abcam) diluted with PBS, to the control antibody sample wells was added with goat F (ab')2 anti-human IgG-Fc (PE) (purchased from Abcam) diluted with PBS, and the plate was incubated at 4°C for 30 min and washed twice with PBS. 100 µL of PBS was added per well to resuspend the cells, which were detected on a CytoFlex (Bechman) flow cytometer to calculate the corresponding MFI.

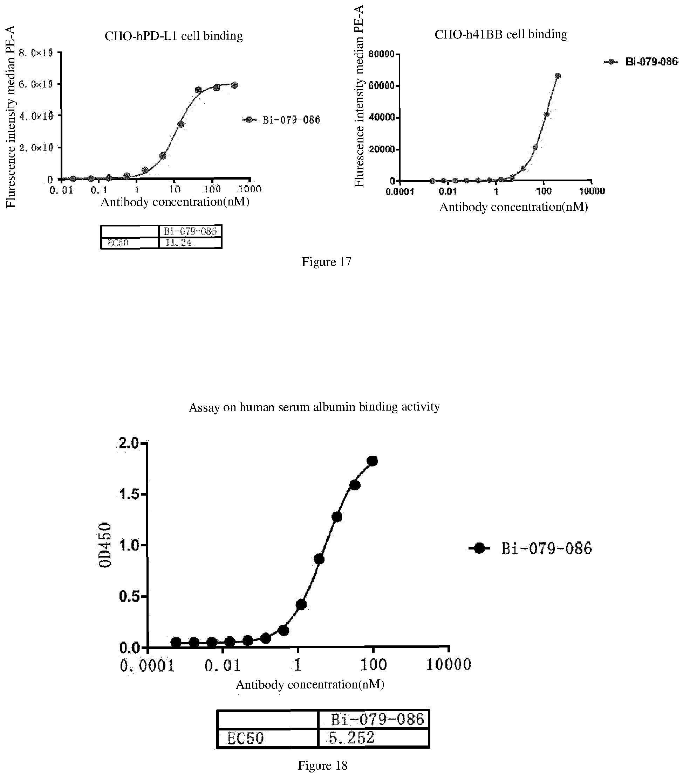

In the assay experiments by the method as described above, as shown in FIG. 17, the results showed that the anti-PD-L1/41BB/human serum albumin trispecific antibodies Bi-79-86 of the present invention had binding activities to both CHO-hPD-L1 cells and CHO-41BB cells.

### 4.4 Determination of binding of anti-PD-L1/41BB/human serum albumin trispecific antibody to human serum albumin at an ELISA level

In this experiment, the binding capacity of the anti-PD-L1/41BB/human serum albumin trispecific antibody Bi-79-86 to the human serum albumin was detected by a method of ELISA reaction, and the experimental method was the same as that in Example 1.3.

In the assay experiment by the method as described above, the results are shown in FIG. 18, and the anti-PD-L1/41BB/human serum albumin trispecific antibody of the present invention can bind to human serum albumin at an ELISA level.

### 4.5 Determination of the ability of anti-PD-L1/41BB/human serum albumin trispecific antibodies to bridge cells expressing PD-L1/41BB

Human PD-L1 and human 41BB were expressed on the surface of tumor cells and immune cells respectively, and the cell bridging experiment proved the capacity of the trispecific antibody Bi-79-86 to pull two cells closer by simultaneously binding to the cell overexpressing human PD-L1 (CHO-hPD-L1) and the cell overexpressing human 41BB (CHO-h41BB), with the specific procedure as follows.

2×10⁷ CHO-hPDL1 and CHO-h41BB cells were respectively taken, centrifuged, resuspended by two dyes, CellTrace^{™} CFSE and CellTracer^{™} Violet BMQC Dye, and incubated for 12 min at 37°C in the dark; Bi-079-Asa 086, A-Na-19, alpha HSA (Ablynx benchmark) were diluted by 2-fold serial dilutions with PBS(starting at 400nM and 12 concentrations in total). CHO-hPDL1 and CHO-h41BB cells were centrifuged, then resuspended with PBS , and mixed at a cell ratio of 1:1. 100 µL/well of the mixture was added to a 96-well plate, and the supernatant was discarded by centrifugation. Human serum albumin was diluted to 2 µg/mL with PBS and then added to the cell plate with 50µL per well, and 50 µL/well of the antibody prepared above was added. The mixture was blended, incubated at 37° C for 2 h in the dark, and detected on a CytoFlex (Bechman) flow cytometer.

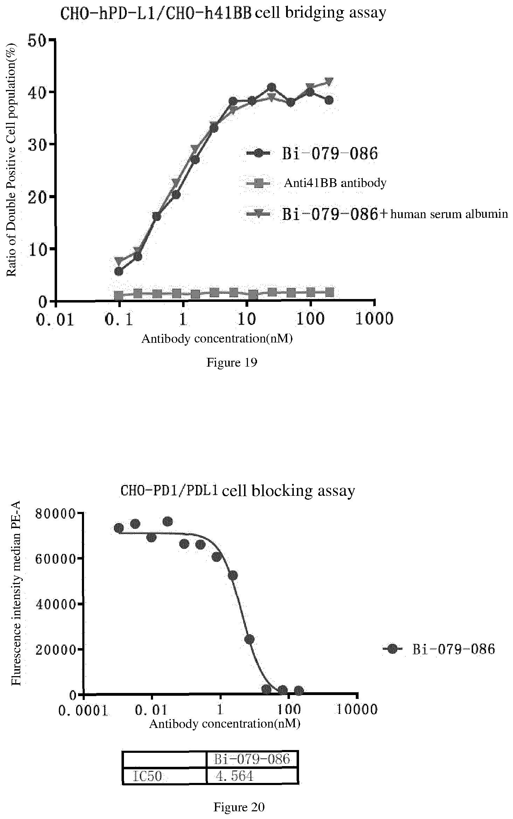

In the assay experiment by the method as described above, as shown in FIG. 19, the experiment results showed that the anti-PD-L1/41BB/human serum albumin trispecific antibody Bi-79-86 of the present invention can pull the CHO-hPDL1 and CHO-h41BB cells closer under the condition that the system contained high concentration of human serum albumin, which proved that Bi-79-86 can simultaneously bind to different antigens expressed on different cell surfaces, and the binding activity was not influenced by the human serum albumin in the system.

### 4.6 Blocking of the binding of human PD-L1 to human PD-1 by anti-PD-L1/41BB/human serum albumin trispecific antibody

The method of detecting the blocking of the binding activity of PD-L1 protein to PD-1 cells by the purified anti-PD-L1/41BB/human serum albumin trispecific antibody Bi-79-86 was the same as that in Example 1.6. In the assay experiment by the method as described above, as shown in FIG. 20, the experiment results showed that the anti-PD-L1/41BB/human serum albumin trispecific antibody Bi-79-86 molecule can block the binding of PD-L1 protein to PD-1 cells.

In summary, the binding activity of the parent antibody can be effectively maintained by linking two different nanobody domains to the N-terminus of the structure of CH1-CL and linking one nanobody domain to the C-terminus of CH1 to form a trispecific antibody In this example, the combination of anti-PD-L1 and anti-41BB nanobody was used to bind to two antigens on different cells, the results indicated that the trispecific antibody structure of the present invention can simultaneously bind to PD-L1 and 41BB at a cellular level and bridge cells expressing PD-L1 and 41BB, respectively, and the binding and bridging activities were not affected by human serum albumin in the system.

### Example 5: Anti-PD-L1/PD-L2 bispecific antibody Fc fusion protein

### 5.1 Construction of anti-PD-L1/PD-L2 bispecific antibody Fc fusion protein

The Fc domain of the antibody can bind to FcRn to prolong the physical half-life of the drug; the Fc domain can bind to other Fc receptors, causing downstream reactions such as ADCC/CDC; the Fc domain can specifically bind to ProteinA, which can facilitate protein purification.

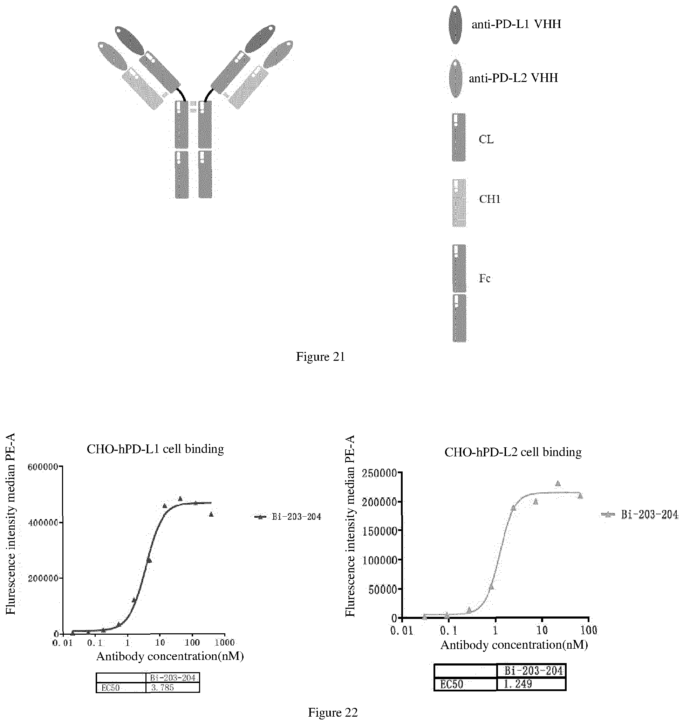

In order to confirm whether the M-Body technique was applicable in the case of fusion with Fc, the present inventors constructed an anti-PD-L1/PD-L2 bispecific antibody designated as Bi-203-204, which contained 2 different polypeptides, with the schematic structure as shown in FIG. 21. Wherein, peptide chain #1 had an amino acid sequence as set forth in SEQ ID NO: 27, which contained anti-PD-L1 nanobody SEQ ID NO: 28, the C-terminus of the nanobody amino acid sequence being directly linked to a CH1 amino acid sequence as set forth in SEQ ID NO: 3 derived from human IgG1; and the peptide chain #1 was obtained by directly linking the human IgG1 (LALAmutation type) Fc (SEQ ID NO: 29) domain to the C-terminus of CH1 region; peptide chain #2 had an amino acid sequence as set forth in SEQ ID NO:30, which contained anti-PD-L2 nanobody HZ-D-NA-96-01 amino acid sequence SEQ ID NO:31, and peptide chain #2 was obtained by directly linking C-terminus of the amino acid sequence to the human kappa light chain constant region (CL).

### 5.2 Expression and purification of anti-PD-L1/PD-L2 bispecific antibody Fc fusion protein

In this example, the nucleotide sequences encoding each of the 2 strands of the anti-PD-L1/PD-L2 bispecific antibody Bi-203-204 constructed in Example 5.1 were ligated into a commercially available eukaryotic expression vector pCDNA3.1(+) via a multiple cloning site and expressed and purified in eukaryotic cells to obtain the bispecific antibody Bi-203-204, with the specific procedure as follows.

The plasmid was transferred into Expi-CHO cells using Expic^{™} Expression System Kit (purchased from Thermo) according to the transfection method described in the product instructions. After 5 days of cell culture, the supernatant was collected and the target protein was purified by protein A magnetic bead (purchased from GenScript) sorting method. The magnetic beads were resuspended in an appropriate volume of binding buffer (PBS +0.1% Tween 20, pH 7.4) (1-4 folds of bead volumes) and then added to the sample to be purified, and the mixture was incubated for 1 h at room temperature by gently oscillating. The sample was placed on a magnetic stand (purchased from Beaver), the supernatant was discarded, and the beads were washed 3 times with binding buffer. The elution buffer (0.1M sodium citrate, pH 3.2) was added in the volume of 3-5 folds of that of the magnetic beads shaken at room temperature for 5-10 min. The sample was placed on a magnetic stand, the elution buffer was collected, transferred to a collecting pipe added with a neutralization buffer (1M Tris, pH 8.54), and the mixture was blended. The purity of the protein was determined by HPLC. HPLC method was as follows: mobile phase: 150 mM Na₂HPO₄•12H₂O, pH 7.0. Chromatographic conditions were as follows: detection wavelength: 280 nm, column temperature: 25°C, flow rate: 0.35 ml/min, detection time: 20 min, Zenix-C SEC-300 column (SEPAX 4.6×300 mm, 3 µm). The experimental results showed that the anti-PD-L1/PD-L2 bispecific antibody Fc fusion protein Bi-203-204 had better purity (>99%), and the purified molecule of Bi-203-204 contained 4 peptide chains and were correctly paired according to the size of the protein molecule obtained by purification.

### 5.3 Determination of antigen binding capacity of anti-PD-L1/PD-L2 bispecific antibody Fc fusion protein

The amplified CHO-hPD-L1/CHO-hPD-L2 cells was adjusted to a cell density of 2×10⁶ cells/mL, 100 µL of the cell was added to each well of a 96-well flow plate, followed by centrifugation for later use. The purified bispecific antibody was diluted by 3-fold serial dilutions with PBS(starting at 400nM and 12 concentrations in total), 100 µL of the diluted sample was added to each well of the 96-well flow plate containing cells, and the plate was incubated at 4°C for 30 min and washed twice with PBS. To each well of the purified antibody sample wells was added with 100 µL of goat F (ab')2 anti-human IgG-Fc (PE) (purchased from Abcam) diluted with PBS, and the plate was incubated at 4°C for 30 min and washed twice with PBS. 100 µL of PBS was added per well to resuspend the cells, which were detected on a CytoFlex (Bechman) flow cytometer to calculate the corresponding MFI.

In the assay experiments performed as described above, as shown in FIG. 22, the results showed that the anti-PD-L1/PD-L2 bispecific antibody Fc fusion protein Bi-203-204 of the present invention had binding activities to both CHO-hPD-L1 cells and CHO-hPD-L2 cells.

In summary, the binding activity of the parent antibody can be effectively maintained by linking two different nanobody domains at the N-terminus of the structure of CH1-CL and linking the human antibody Fc domain at the C-terminus of CH1 to form the bispecific specific antibody Fc fusion protein. At the same time, the Fc domain can facilitate the purification of the antibody and the formation of a stable tetramer structure.

All documents mentioned in this application are incorporated by reference in this application as each individually incorporated by reference. Furthermore, it should be understood that various changes or modifications of the present invention can be made by those skilled in the art after reading the above teachings of the present invention, and these equivalents also fall within the scope of the appended claims of the present application.

## Claims

1. A method for construction of a multispecific antibody, **characterized by** comprising the steps of:
(i) constructing a first polynucleotide encoding a first polypeptide having a structure represented by Formula I from N-terminus to C-terminus and a second polynucleotide encoding a second polypeptide having a structure represented by Formula II from N-terminus to C-terminus, respectively,
A1-L1-B1-L2-CL-L3-A2 (Formula I)
A3-L4-B2-L5-CH1-L6-A4 (Formula II)
wherein, A1, A2, A3, and A4 are each independently an antibody or antigenic fragment thereof that targets a target of interest, and the target antigens targeted by each of A1, A2, A3, and A4 can be the same or different;
L1, L2, L3, and L4 are each independently a null or linker element;
B1 and B2 are both null, or B1 and B2 are the VL and VH regions, respectively, of an antibody targeting the same target;
and a disulfide bond may be formed between the CL region of the first polypeptide and the CH1 region of the second polypeptide, such that the antibody has a heterodimeric form; and
(ii) expressing the first polynucleotide and the second polynucleotide to obtain the first polypeptide and the second polypeptide, and dimerizing the first polypeptide and the second polypeptide to form a multispecific antibody with a heterodimeric form.

2. A multispecific antibody, **characterized in that** the antibody comprises a first polypeptide represented by Formula I from N-terminus to C-terminus and a second polypeptide represented by Formula II from N-terminus to C-terminus,
A1-L1-B1-L2-CL-L3-A2 (Formula I)
A3-L4-B2-L5-CH1-L6-A4 (Formula II)
wherein,
A1, A2, A3, and A4 are each independently an antibody or antigenic fragment thereof that targets a target of interest, and the target antigens targeted by each of A1, A2, A3, and A4 can be the same or different;
L1, L2, L3, and L4 are each independently a null or linker element;
B1 and B2 are both null, or B1 and B2 are the VL and VH regions, respectively, of an antibody targeting the same target of interest;
and a disulfide bond may be formed between the CL region of the first polypeptide and the CH1 region of the second polypeptide, such that the antibody has a heterodimeric form.

3. A fusion protein, **characterized in that** the fusion protein comprises the multispecific antibody of claim 2, and the first polypeptide of the multispecific antibody has a structure represented by Formula III from N-terminus to C-terminus,
A1-L1-CL-L3-Fc (Formula III)
wherein, Fc is a Fc fragment of an antibody, comprising a CH2 domain and a CH3 domain;
and the fusion protein may form a homodimer via disulfide bonding between Fc fragments.

4. An isolated combination of polynucleotides, **characterized by** comprising a first nucleotide and a second nucleotide, wherein the first nucleotide encodes a first polypeptide of the multispecific antibody of claim 2 or of the fusion protein of claim 3, and the second nucleotide encodes a second polypeptide.

5. A vector, **characterized by** comprising a combination of polynucleotides of claim 4.

6. A host cell, **characterized by** that said host cell comprises a vector of claim 5, or have incorporated into its genome a combination of polynucleotides of claim 4;
alternatively, the host cell expresses a multispecific antibody of claim 2 or a fusion protein of claim 3.

7. A method of producing an antibody, **characterized by** comprising the steps of:
(a) culturing a host cell of claim 6 under suitable conditions to obtain a culture comprising a multispecific antibody of claim 2 or a fusion protein of claim 3; and
(b) purifying and/or isolating the culture obtained in step (a) to obtain the antibody.

8. An immunoconjugate, **characterized by** comprising:
(a) a multispecific antibody of claim 2 or a fusion protein of claim 3; and
(b) a coupling moiety selected from the group consisting of: a detectable label, a drug, a toxin, a cytokine, a radionuclide, or an enzyme, a gold nanoparticle/nanorod, a nano magnetic particle, a viral coat protein or VLP, or a combination thereof.

9. Use of a multispecific antibody of claim 2, a fusion protein of claim 3, or an immunoconjugate of claim 8 in the manufacture of a medicament, a reagent, a detection plate, or a kit;
wherein the reagent, the detection plate or the kit is used for detecting the presence or absence of the target molecule of interest in the sample;
and the medicament is used for treating or preventing tumors expressing target molecules of interest.

10. A pharmaceutical composition, **characterized by** comprising: (i) a multispecific antibody of claim 2, a fusion protein of claim 3, or a immunoconjugate of claim 8; and (ii) a pharmaceutically acceptable carrier.
